(19) Europäisches Patentamt
European Patent Office
Office européen des brevets

(11) Publication number: **0 338 459**
**A2**

(12) # EUROPEAN PATENT APPLICATION

(21) Application number: **89106755.5**

(51) Int. Cl.⁴: **A61K 7/06**

(22) Date of filing: **14.04.89**

(30) Priority: **18.04.88 US 182784**

(43) Date of publication of application:
**25.10.89 Bulletin 89/43**

(84) Designated Contracting States:
**AT BE CH DE ES FR GB GR IT LI LU NL SE**

(71) Applicant: **Trustees of Boston University**
**881 Commonwealth Boulevard**
**Boston Massachusetts 02215(US)**

Applicant: **Angio-Medical Corporation**
**1345 Avenue of the Americas**
**New York New York 10105(US)**

(72) Inventor: **Solomon, Henry**
**112 East 74 Street**
**New York New York 10021(US)**
Inventor: **Catsimpoolas, Nicholas**
**65 Montvale Road**
**Newton Center Massachusetts 02159(US)**
Inventor: **Mirsky, Jan**
**103 East 75th Street**
**New York New York 10021(US)**
Inventor: **Kamarei, Ahmad Reza**
**40 Vine Street**
**Lexington Massachusetts 02173(US)**
Inventor: **Klein, Jonathan D.**
**8016 Fairfield Way**
**Commack New York 10021(US)**
Inventor: **Bishop, Michael A.**
**4011 Hopevale Avenue**
**Sherman Oaks California 91403(US)**
Inventor: **Sinn, Robert S.**
**248 West 88th Street**
**New York New York 10023(US)**

(74) Representative: **Schulze Horn, Stefan, Dipl.-Ing.**
**Goldstrasse 36**
**D-4400 Münster(DE)**

(54) **Composition for promoting hair growth in androgenetic alopecia and method thereof.**

(57) Composition and method for promoting new hair growth and preventing hair loss in mammals exhibiting common baldness pattern is described. In particular, the composition contains isolated omentum lipid material, a preservative, an emulsifier, an antioxidant and a skin filler incorporated in various formulations including lotions, creams, ointment, gels, sprays or stick.

EP 0 338 459 A2

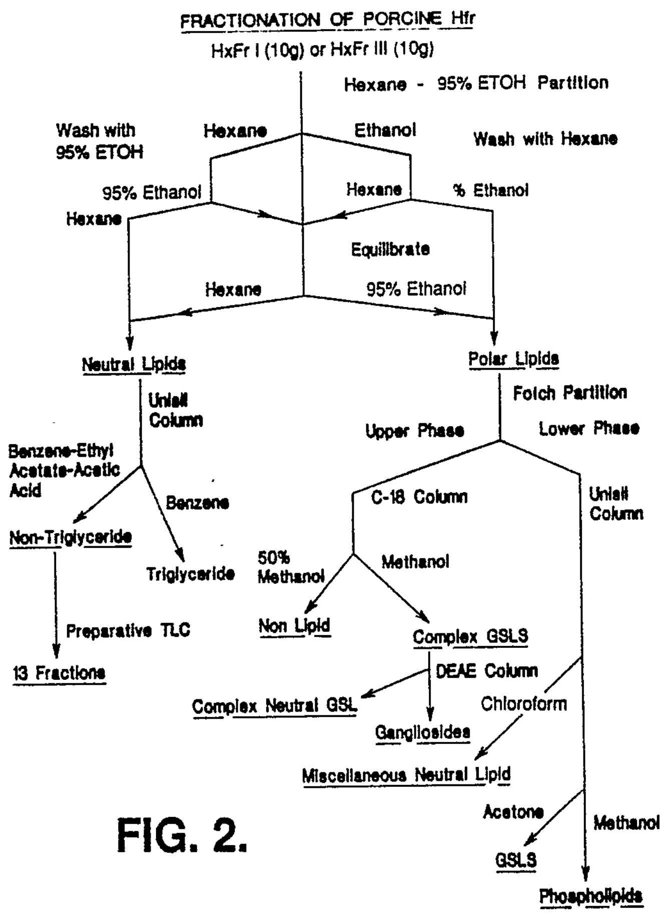
FRACTIONATION OF PORCINE Hfr
HxFr I (10g) or HxFr III (10g)
Hexane - 95% ETOH Partition
Wash with 95% ETOH
Hexane
Ethanol
Wash with Hexane
95% Ethanol
Hexane
% Ethanol
Hexane
Equilibrate
Hexane
95% Ethanol
Neutral Lipids
Unisil Column
Benzene-Ethyl Acetate-Acetic Acid
Benzene
Non-Triglyceride
Triglyceride
Preparative TLC
13 Fractions
Polar Lipids
Folch Partition
Upper Phase
Lower Phase
C-18 Column
Unisil Column
50% Methanol
Methanol
Non Lipid
Complex GSLS
DEAE Column
Complex Neutral GSL
Gangliosides
Chloroform
Miscellaneous Neutral Lipid
Acetone
Methanol
GSLS
Phospholipids

FIG. 2.

# COMPOSITION FOR PROMOTING HAIR GROWTH IN ANDROGENETIC ALOPECIA AND METHOD THEREOF

## CROSS REFERENCE TO RELATED APPLICATION

This application is a continuation-in-part of U.S. Patent application Serial No. 046,909, filed May 5, 1987, which in turn is a continuation-in-part of U.S. patent application Serial No. 948,017, filed on December 31, 1986, now abandoned.

## FIELD OF THE INVENTION

This invention relates to a composition and the method thereof for stimulating new hair growth in mammals. In particular, a composition containing isolated mammalian omentum lipids is used to prevent hair loss and stimulate new hair growth in mammals exhibiting common pattern baldness.

## BACKGROUND OF THE INVENTION

Common pattern baldness or androgenetic alopecia occurs in both sexes in mammals. Alopecia androgenetica (i.e., androgenic alopecia or common pattern baldness) is a familial disease inherited as an autosomal dominant trait and, as its name suggests, circulating androgens are thought to be necessary to cause the hereditary alopecia to develop.

Whereas there are historical instances where baldness has been desirable there are many more cases where persons exhibiting traits of baldness have attempted to prevent hair loss or camouflage this condition.

The patent literature contains numerous examples of compositions and claims for promoting hair growth. One of these compositions is applied in cream form and contains principally marrow, such as beef marrow, with additions of bergamot oil, alcohol, oil and sulfur as described in U.S. Patent No. 4,520,012 issued on May 28, 1985 to Alfonsi. The patent teaches that the composition enhances blood circulation in the scalp dermis to nourish the hair, possibly due to the effects of the bergamot oil. It is noted, however, that bergamot oil is known to be a skin irritant and Alfonsi also warns that more than 5% bergamot oil in the composition can actually cause hair loss. (Alfonsi, col. 2, lines 6-12).

Another hair growth composition was described in U.S. Patent No. 4,503,047 issued on March 5, 1985 to Banfi. This composition contains horse radish extract in combination with allyl isothiocyanate which is believed to stimulate the forming ability of the hair bulbs. Additional elements of the composition include rhodanide ions, copper ions and sulfur containing amino acids.

The pharmaceutical composition under the generic name Minoxidil and marketed by Upjohn Company of Kalamazoo, Michigan is disclosed as a promotion of hair growth in U.S. Patent No. 4,139,619 issued on February 13, 1979 to Chidsey. The drug is claimed to increase the rate of terminal hair growth (the broad diameter and colored hair that is readily seen) and to convert vellus hair (fine, colorless hair) to terminal hair.

Minoxidil is a potent antihypertensive substance which has been documented in studies to cause hair growth in virtually all patients that were orally treated with the drug for more than one year. de Villez, R. L., Arch. Dermatol. 121: 197-202 (1985). However, within four months of discontinuing the Minoxidil treatment all of the newly grown hairs had fallen out. Id. at 197.

Patterns of hair loss are classified in the now universally accepted system described in Hamilton, James B. Annals of N.Y. Acad. of Sci. 53: 708-628 (1951). Type I represents a non-balding scalp; type II - a bitemporal recession; type III - border line balding or thinning hair; types IV-V represent slight balding and types VI-VIII represent horse shoe outlined baldness, types VII and VIII representing the most advanced stages of baldness.

Preliminary results indicate that about one-third (1/3) of the subjects treated with Minoxidil produced cosmetically acceptable hair regrowth, and those patients having less severe baldness (i.e., Hamilton Types III-VI) showed the greatest amount of regrowth.

Minoxidil studies involved about 2,000 healthy subjects with male pattern baldness and, during the first four months of a twelve month study, randomly allocating to patients a 2% Minoxidil lotion or a placebo under double-blind conditions. Patients applied the lotions twice topically daily. Clissold, et al., Drugs 33: 107-122 (1987).

Studies involving smaller numbers of patients but using the identical protocol as described in Clissold, supra have been published in Olsen et al., J. of the Amer. Acad. of Derm. I5: 30-37 (I986); Olsen et al. J. of the Amer. Acad. of Derm. I3: I85-I92 (I985); and de Villez, R., Arch. of Derm. I2I: I27-202 (I985).

Other studies involving 2 and 3% Minoxidil using a similar protocol have been reported in Shupack et al., J. of the Amer. Acad. of Derm I6: 673-6 (I987); de Villez, R. J. of the Amer. Acad. of Derm. I6: 669-72 (I987); and Rietchel et al., J. of the Amer. Acad. of Derm. I6: 677-85 (I987).

From these dose-response studies, it has been found that the best new hair growth were produced upon use of the 2% Minoxidil lotion than any other dosage. Clissold, supra at page I08-I09.

Based on the results of these reported studies, the present inventors created a predictor of hair growth described as the SinnTM plot predictor to compare results of the inventive studies against those of the Minoxidil studies. The SinnTM plot predictor and the resulting comparative studies are disclosed in the Detailed Description, infra and show that the inventive composition achieves a greater increase in hair regrowth earlier in the treatment (i.e. the initial four months) than the hair growth results achieved with the 2% Minoxidil lotion.

Given the statements by some investigators that topical Minoxidil is more effective in diminishing the rate of hair loss than in restoring already bald areas, that the effect of Minoxidil on hair growth is reversed once the treatment has been stopped and that there is uncertainty as to systemic effects of the topical Minoxidil treatment, it is advanced that the inventive composition achieves higher percentage of hair regrowth while at the same time provides an improved means for treating common pattern baldness without an probability of harmful side effects. See Stern, R., Arch. Derm. I23: 62-65 (I987).

The average adult scalp has approximately 3,000 hair follicles per square inch. Id. at 64. The greatest increases in the number of terminal hair regrown after a year of Minoxidil therapy was reported in Olsen, supra at page 37, in which an average of 365 terminal hairs per square inch were grown after using the 3% Minoxidil lotion. Olsen's work indicates that treatment with Minoxidil requires a long period before therapeutic efficiency can be judged. For example after only four months, Minoxidil treated patients had increased only about 96 terminal hairs per square inch more than placebo-treated patients. Olsen '86, supra at 31.

At current prices, the cost of treating a patient for a year of therapy with Minoxidil is about $7I5.00. Stern, supra at page 65. This cost does not reflect the dermatology bills for diagnosis and the prescription. Treatment costs are particularly high when considering that the use of the topical Minoxidil may be prophylactic only in preventing hair loss, rather than promoting new hair growth.

Additional research in the field of hair growth has investigated the topical application of vitamin A or all-trans-retinoic acid (tretinoin), alone and in combination with 0.5% Minoxidil, to study the effects on hair growth. Bazzano, et al., J. of the Amer. Acad. of Derm. 15: 880-893 (I986). Of the subjects who received tretinoin alone, most of the hair growth was of the lanugo type (prenatal hair in mammals).

It has been demonstrated by Michael I. Klibaner, M.D., Ph.D. of Angio-Medical Corp., Pathobiology Laboratory in Boston, Massachusetts, that vitamins A and D inhibit angiogenesis in Chick Embryo Chorioallantoic Membrane Assays ("CAM assays") indicating that the topical application of vitamins alone to hair follicles may exert a slightly negative effect in new hair growth.

Therefore, a relatively inexpensive and nonirritating cosmetic composition which promotes new growth of terminal hair has been unknown in the art until the present invention. It has been unexpectedly shown that a composition containing isolated omentum lipids in combination with sodium hyaluronate and other ingredients, promotes new hair growth in mammals.

The term "new hair growth" refers to new hair grown in balding areas genetically programmed for hair follicles that produce terminal hairs, as opposed to merely lengthening already grown hair. The term also refers to terminal hair, or the hairs of the scalp having the larger diameter and being pigmented, and indeterminate hairs defined as pigmented hairs which are thinner and shorter than terminal hairs, as opposed to vellus hair, the fine colorless hairs.

It is thus an object of the present invention to provide a method and a composition for substantially preventing hair loss in mammals and to promote new hair growth in within weeks of topical treatment to balding areas.

## SUMMARY OF THE INVENTION

Topical application of the composition including omentum lipid material promotes new terminal hair growth, while at the same time preventing further hair loss in the progression of androgenetic alopecia.

The composition may be applied to the skin in the form of lotions, ointments, gels, sticks, or creams, although lotion form is preferable. Applying the composition to balding or bald areas of mammalian scalp has been observed to stop further hair loss and to promote new hair growth of terminal hairs to enhance hair distribution.

## BRIEF DESCRIPTION OF THE DRAWINGS

Figure 1 is a diagrammatic flow chart of the method for obtaining hexane omentum extracts.

Figure 2 is a flow chart diagramming the fractionation of porcine HxFr.

Figure 3 is a photograph of the vertex of the head of a test subject exhibiting Hamilton type VI male pattern baldness prior to treatment with Omexin™ hair lotion.

Figure 4 is a photograph of the same area of the subject's head of Figure 3 after six months of treatment with Omexin™ hair lotion, according to the invention.

Figure 5 is a photograph of a vertex of a second test subject's head exhibiting Hamilton type VI male pattern baldness prior to treatment with Omexin™ hair lotion, according to the invention.

Figure 6 is a photograph of the same area of the test subject's head of Figure 4 after six months of treatment with Omexin™ hair lotion.

Figure 7 is a graph illustrating the Sinn™ plot predictor for hair growth for one year calculated from Olsen '85 and Reitschel, supra. Data obtained from these inventors' test results is superimposed on the graph illustrating the greater increase of hair growth earlier in the treatment period as compared to the 2% Minoxidil treatment.

Figure 8 is a graph of the increase in new hair growth achieved by test subjects over a one year period after using a 2% Minoxidil treatment according to the reported results of Shupack, Reitschel, Olsen '85, de Villez, and Olsen '86 discussed infra.

Figure 9 is a graph representing the increase of total new hair growth after eight months of treatment with Omexin™ hair lotion on 19 subjects, as compared to the subjects' baseline data.

Figure 10 is a graph illustrating the decrease in bald spot diameter sizes after eight months of treatment with Omexin™ hair lotion.

## DETAILED DESCRIPTION OF THE PREFERRED EMBODIMENTS

### I. Omentum Material - Omexin™ hair Lotion

The principal ingredient of the inventive composition is isolated omentum lipid, preferably extracted from mammals. The omentum material may be applied in conventional cosmetic forms, such as ointments, lotions, paste, gels, aerosols, sticks and the like which are known in the art.

The preferred embodiment is a lotion form which combines the omentum material with emulsifiers, antioxidants, hygroscopic agents, chelating agents, skin fillers, silicon and preservatives. When applied to areas genetically coded for hair growth, the novel composition promotes new hair growth and prevents further hair loss in the progression of alopecia.

Omentum material may be extracted by chloroform-methanol from feline omentum as described in Goldsmith, et al., J. Amer. Med. Assoc., 252: 2034-36 (1984).

Omentum materials are used in wound healing as described in copending U.S. Patent Application, Serial No. 805,206, filed on December 4, 1985 and entitled "Method for Enhancement of Healing of Epithelial Wounds in Mammals" by Catsimpoolas, et al., herein incorporated by reference.

These omentum materials are also used in angiogenesis for myocardial conditions such as myocardial infarctions, angina, vascular or coronary implants and angioplasty as described in copending U.S. Patent Application, Serial No. 811,375, filed on December 20, 1985 and entitled "Method for Treatment of Angina and Myocardial Infarctions with Omentum Lipids" by Catsimpoolas, et al., herein incorporated by reference.

It is also noted that copending U.S. Patent Application Serial No. 811,894, filed on December 20, 1985 entitled "Method for Healing Bone Damage and Composition" by Lane, et al. shows bone healing use of omentum materials, this application is hereby incorporated by reference.

### (Ia) Preparation of the Lipid Extract from Omentum by Brittle Grinding and Hexane Extraction.

The preferred method for preparation of omentum for use in the hair growth composition is a brittle grinding technique (also known as a cryogenic technique) combined with direct hexane extraction of the thus obtained omentum powder. Porcine omentum is preferred for use in the composition, however, bovine, feline, or any other type of mammalian omentum may be used.

The porcine omentum is cut or broken into pieces. Freezing the omentum is accomplished by a method described in copending U.S. Patent Application, Serial No. 8ll,507, filed on December 20, l985 by Kamarei, et al. entitled "Brittle Grinding and Extraction of Animal and Plant Derived Material"; herein incorporated by reference.

The omentum material is frozen to lower its temperature to an empirically critical "Brittleness Temperature" which converts the unbreakable, viscous, and sticky material into an extremely brittle and fragile substance.

Freezing can be accomplished by various methods, including:

l. Air Freezing:
  (a) Blast freezing
  (b) Fluidized-bed freezing

2. Plate Freezing
3. Liquid-immersion Freezing and
4. Cryogenic Freezing (freezant undergoes a change of state):
  (a) Liquid nitrogen ($LN_2$), - l96° C (77K)
  (b) Subliming carbon dioxide (dry ice), -79° C (l94K)
  (c) $CCl_2F_2$ (refrigerant Freon -l2), -30° C (243K)

Cryogenic freezing with $LN_2$, among all the above methods, is the most desirable method.

(lb) Cryogrinding Treatment

Upon obtaining the brittle pieces of tissues, they may be transferred to any size reduction equipment and homogenized (ground) for the desired length of time, i.e., a few minutes at 22,000 RPM. At larger scales, roll mills with both attrition and impact grinding could be used. It is important that the tissue be kept at or below its brittle temperature throughout the grinding process. Grinding below the brittleness temperature is required in order to produce the necessary small particle size. For this purpose one could occasionally add, if needed, quantities of $LN_2$ to the size-reduction equipment, provided that there is a vent to allow exhaustion of the nitrogen vapor generated during the process.

Upon completion of the process, an extremely fine, and free-flowing (non-sticky) cryoground tissue ("tissue powder") results. The resultant powder obviously may contain some granular lumps as well as fine indiscrete particles.

Parallel to the tremendous increase of surface area (i.e., ratio of surface area to volume), there is also an inherent reduction of density. As an example, density of omentum powder is 0.44 (±5%) g/ml which is almost half of the density of lipids extracted from omentum.

(lc) Cryosieving of Tissue Powder

The resultant cryoground tissue powder, while extremely find, is not "uniform" with regard to size. Consequently, if uniformity in a particular size range is desired for the following steps, tissue powder should be sieved at or below its brittleness temperature. For this purpose, one may use stacked stainless steel standard sieves having standard designations.

The best uniform fraction of omentum powder is in the range of l50 to 600 um (micrometers = um).

The uniform tissue powder can be further processed, transferred, or stored at regular freezing temperature (e.g., -18° C = 0° F). However, even at regular freezing temperature some chemical changes (e.g., oxidation of unsaturated lipids, especially because of tremendous surface area generated by cryogrinding; insolubilization or destabilization of proteins; and degradation of pigments and vitamins and other biomolecules) may slowly occur. Reduction of freezer temperature will cause decline of the rates of the above reactions. Consequently, for longer storage times, it is recommended that the final tissue powder be stored at -40° C, under vacuum or inert gas, and in the dark, (to prevent any possible photo-catalytic reactions). Preliminary evaluation shows that various tissue powders stored at -40° C for up to 2 months, did not show any physical changes (texture, color, odor, etc.) in the product.

To use the uniform cryoground product, one should desirably "thaw" the tissue powder. Since thawing of non-fluid tissues is inherently slower than freezing, when comparable temperature differentials are employed (due to different thermal properties of ice vs. water), tissue powders may be subject to damage by chemical or physical (and less microbial or enzymatic) means. In light of these considerations, one skilled in the art will recognize that the thawing process must be carefully considered.

(Id) Direct Hexane Extraction of Omentum Powder

By the general process illustrated in Fig. 1, 200 kg. of cryoground porcine omentum powder was warmed up from -10 °C to room temperature and added to 600 liters of hexane. Another 600 liters of hexane was added to the mixture and allowed to settle overnight in a tank (i.e. Tank 1) at 20 °C. The aqueous phase was drained from the tank bottom and Celite 545 was added to the tank and heated to 30 °C. The mixture was mixed and passed through a horizontal filter press to a second tank (i.e. Tank 2) where it was allowed to settle for one hour. The aqueous phase was again drained from tank 2. Hexane was partially evaporated from tank 2 at 30 °C under vacuum.

Continued evaporation of hexane (plus any residual water) was carried out and the mixture was allowed to settle overnight at room temperature.

Evaporation of the hexane was completed under vacuum and nitrogen was introduced from the bottom of tank 2 while increasing the temperature to 60 °C. Omentum extract was collected and frozen until ready for use in the manufacturing process of Omexin™ hair lotion.

(Ie) Chloroform/Methanol Extraction of Omentum Powder

500 g. uniform porcine omentum powder was warmed up to room temperature and extracted with 10 times chloroform/methanol (2:1, v/v) in a glass blender (22,000 RPM, 30 seconds). The solvent extract was centrifuged (2,000 RPM, 20 minutes) and subjected to rotary evaporation (under vacuum, 37 °C) until dryness, i.e., neither any solvent condensation occurs, nor any solvent odor is present. A whitish chloroform/methanol fraction (CMFr) weighing 388 g. (i.e., 77.6%) was obtained. The CMFr could be further subjected to hexane/ethanol fractionation.

(If) Supercritical $CO_2$ Extraction of Omentum Powder

1905.4 g. uniform porcine omentum powder was extracted with super-critical (SC)-$CO_2$ in four trial batches. The solvent to feed ratio was 300 (1 lb./min.) was used. A total of 12 extract fractions were collected from each run. The extractor was maintained at 37 °C and 3500 psi, first separator at 40 °C and 1500 psi, and second separator at 29-30 °C and about 1000 psi, where the bulk of extract was collected. The solvent from the second separator was fed into a knock-out vessel at about 1500 psi and then recycled. The residue in the extractor was in the range of 8-17% of the reed. It was interesting to find out that SC-$CO_2$ extracts about 83-92% of the feed which is more than chloroform/methanol (with traditional homogenization practice). This would suggest that cryogrinding prior to extraction is indeed effective.

(Ig) Mild Thermal Extraction of Omentum Powder

In order to thermally extract lipids from omentum or any other fatty tissue, it is necessary to: (1) melt or liquidify lipids, (2) separate melted lipids from the rest of the tissue, and (3) filter the resultant oil or fat from residues. All above steps were combined as "one step" as follows:

1000 g. uniform porcine omentum powder was placed in two stainless steel 150 um (micrometers = um) (#100) sieves. These sieves were stacked on the top of 38 um (#400) sieves and collecting pans. The stacks were placed in an oven with mild heat (70 °C.) Upon gradual thawing of omentum powder, lipids became gradually melted while proteinaceous materials became denatured. This resulted in the shrinkage and final collapse of the powdery structure of omentum powder. Melted lipid, however, easily passes through a 150 um sieve and fell as droplets on the 38 um sieve, where it was filtered and separated from small residue particles, and finally clear oil was collected in the collecting pans. The total thermal extraction time was 3 hours, during which occasional mixing occurred. Total recovery was found to be 709.5 g. (i.e., 73.6%). It is notable that above recovery does not cover the compression of the remaining oily materials on the top sieve.

(Ih) Analysis of Preferred Porcine Omentum Extract Used In OmexinTM Hair Lotion

Two samples of porcine omentum were prepared by the preferred brittle grinding technique followed by hexane extraction as discussed in section (Id) supra. Also see McCluer, R., et al, Lipids, (May 1987) reporting feline omentum extract characterization. Similar techniques are performed on porcine omentum lipids.

Two porcine hexane extracts (known as HxFrI and HxFrIII) were fractionated by a method diagrammed in Figure 2 and described infra.

Ten grams of each hexane preparation (HxFrI and HxFrIII) was partitioned between hexane and 93% ethanol and each phase was washed with fresh opposite phase. The washes were then equilibrated with each other and all like phase were combined as illustrated in Figure 2.

Neutral lipids obtained from the evaporation of the hexane were next fractionated on a silica gel column, such as a Unisil column. A large quantity of triglycerides was eluted with benzene; then minor neutral lipids were eluted with a benzene/ethyl/acetate/acetic acid mixture. Fatty acids of the triglycerides were analyzed by gas chromatography (GC) and mass spectrometry (MS) based on methyl esters obtained by alkaline methanolysis.

Polar lipids obtained from the ethanol phase were partitioned according to the Folch method following Folch et al., J. Biol. Chem. 226: 497-509 (1957).

The extraction of omentum by the Folch method is also described in copending U.S. patent application, serial no. 811,505 filed on December 20, 1985 by Catsimpoolas et al and entitled "Lipids from Omentum and Methods for Cosmetic Use", herein incorporated by reference (i.e., the fraction was dissolved in chloroform/methanol (2:1, 20 volumes, v/w) and 0.2 volumes of water were added. Phases were thoroughly mixed, and allowed to separate.)

Water was added to separate the chloroform methanol mixture into two phases. The lipids in the upper phase were absorbed onto a C-18 reverse phase cartridge, eluted with methanol, acidic lipids (i.e., gangliosides) and neutral lipids (i.e., complex glycolipids) and separated on a DEAE - Sephadex column.

The Folch lower phase lipids were run on a Unisil chromatography column and the following three fractions were obtained:

(1) minor neutral lipids eluted with chloroform;
(2) glycolipids eluted with acetone and methanol; and
(3) phospholipids eluted with methanol.

Each fraction was dried, weighed and analyzed by thin layer chromotography (TLC).

Unisil Fractionation of the Hexane Phase Lipids

Benzene Fraction

The triglyceride fraction eluted by benzene constituted about 97% of the first hexane extract (HXFrI). Fatty acids of the triglyceride fraction were analyzed by GC and GC/MS and the percentage distribution of the major components was found to be:

| | |
|---|---|
| 16 : 1 | (2%) |
| 16 : 0 | (24%) |
| 18 : 2 | (10%) |
| 18 : 1 | (30%) |
| 18 : 0 | (16%) |

The remaining ten (I0) percent consisted of I4:0 and fatty acids that were present at less than I% of the total.

Benzene and Ethylacetate Fraction

The minor neutral lipids were further fractionated by preparative thin layer chromatography (TLC) into I3 components. The major components of the minor neutral lipids were free fatty acids, cholestrol and diglycerides in the approximate ratio of I:I:0.5, as estimated by the TLC charring. The remaining components were present in trace amounts identified as various diglyceride molecular species determined by direct probe mass spectrometry and fatty acid analysis. Most of the monoglycerides distributed into the ethanol phase were seen in the chloroform fraction but were eluted from the Unisil column.

## Analysis of Ethanol Phase Lipids

Unisil Fractionation of Folch Lower Phase Extraction

Chloroform Fraction

A chloroform fraction of approximately 0.44% by weight of the total HXFrl fraction was obtained and analyzed by TLC. This fraction was found to contain the same neutral lipids found in the hexane layer discussed supra.

Cholesterol and free fatty acids make up about 5I% of this fraction; monoglycerides represent I2%; diglycerides represent 4%; and triglycerides represent 26%.

Acetone Fraction

Neutral glycolipids in the acetone fraction were determined by TLC analysis to represent less than .00I% of the HxFrl extract.

Approximately 50% of the glycolipids was monoglycosyl ceramide; 30% was diglycosyl ceramide; and 20% was triglycosyl ceramide. The glycolipids in this fraction were only 0.05% of the total fraction weight. Several non-polar components were present in this fraction which represent the majority of the fraction weight.

Methanol Fraction

The phospholipid content of the HxFrl was approximately 0.0I%. Two major components of this fraction were identified as phosphatidylcholine (about 80% of the fraction) and sphingomyelin (about 20% of the fraction).

Folch Upper Phase DEAE Fractions

Neutral Fraction

The neutral fraction of the HxFrl extract sample is less than 0.00I% of the total HxFrl, and contains globosides and three other polyglycosyl ceramides. The TLC mobilities are similar to asialo-GMI, Forssman glycolipid and SSEA-I antigen. ·

The glycolipids of this neutral fraction represent about I6% by weight. The majority of this fraction is phospholipids, which is partitioned into the upper phase with the gangliosides and polyglycolyl ceramides.

Acidic Lipid Fraction

Gangliosides represent less than 0.00l% of the total HxFrl extract in this fraction as analyzed by TLC. This fraction contains 4 major gangliosides in the following descending order: GDla, GM3, GM1 and GD3.

Composition of HxFrlll

The second hexane extract sample (HxFrlll) composition was found to be almost identical to the composition of the first sample, except a significant amount of volatiles, probably moisture, was found in the initial preparation.

The amount of l8:2 fatty acid in the triglyceride fraction (5%) was decreased and the quantity of the polar lipids (i.e., phospholipids and glycolipids) was almost doubled as determined from fraction weights and TLC analysis. The ganglioside content of HxFrlll was estimated to be 0.00l%.

See table 1 infra for a composite of the weights and percentage compositions found during fractionation of the HxFrl and HxFrlll extracts.

TABLE 1

WEIGHTS AND % COMPOSITIONS FOUND DURING FRACTIONATION OF HxFr-I and HxFr-III

(Refer to Fig. 2 for identiy of fraction)

| | HxFr-I | | HxFr-II | |
|---|---|---|---|---|
| FRACTION | Weight gm | % HxFr | Weight gm | % HxFr |
| HxFr | 9.99 | 100 | 10.34 | 100 |
| Neutral Lipds (Hexane phase) | 9.91 | 99.1 | 10.01 | 96.7 |
| Triglycerides | 9.78 | 97.8 | 9.25 | 89.4 |
| Non-triglyceride neutral lipids | 0.12 | 1.2 | 0.14 | 1.2 |
| Polar Lipids (ethanol phase) | 0.077 | 0.77 | 0.055 | 0.53 |
| Folch upper phase | 0.004 | 0.04 | 0.007 | 0.07 |
| Complex GSLs | * | * | 0.004 | 0.04 |
| Complex neutral GSLs | * | * | 0.002 | 0.02 |
| Gangliosides | * | * | 0.002 | 0.02 |
| Folch lower phase | 0.060 | 0.60 | 0.053 | 0.51 |
| Minor neutral lipids | 0.044 | 0.44 | 0.039 | 0.38 |
| GSLs | 0.007 | 0.07 | 0.002 | 0.02 |
| Phospholipids | 0.004 | 0.04 | 0.005 | 0.05 |

* Less than 0.001 gm

(2a) Preparation of the porcine and bovine CMFr

As described in serial no. 811,505 filed on December 20, 1985 by Catsimpoolas, et al. and herein incorporated by reference, for preparation of the porcine and bovine Chloroform Methanol Fraction (CMFr), fresh and/or frozen omentum was obtained from a local slaughterhouse. Omenta tissues were washed with minimum amount of distilled water, weighed and cut into 1-2 g pieces under sanitary conditions. Upon pooling and mixing of omenta pieces, they were divided into 500 g portions. Extra portions were kept frozen at -25 °C.

Each 500 g portion was homogenized with four volumes phosphate buffered saline (PBS) in a glass blender at 22,000 RPM and for 2 min. The resultant homogenate was centrifuged (4000 RPM, 10 min.,

5° C), and the lipid-containing cake was collected (about 98% of omentum weight). The lipid cake was extracted with 10 volumes of chloroform/methanol (2:1) in a glass blender (22,000 RPM, 30 s). The organic solvent containing lipid was centrifuged (2,000 RPM, 20 min., 5° C). Although most of the tissue residue was precipitated as pellet, some finer particles were still suspended after centrifugation. Consequently, attempts were made to collect the solvent phase with minimum tissue residues.

The solvent extract was subjected to rotary evaporation (under vacuum, 37° C) until dryness, i.e., neither any solvent condensation occurs, nor any solvent odor is present. The whitish CMFr weighing 70±2% of the omentum was then collected.

(2b) Preparation of the porcine and bovine HxCMFr

The chloroform/methanol fraction was warmed up to 37° C. Four volumes 95% EtOH (obtained from absolute ethanol) and six volumes hexane was added to the CMFr and mixed for 20 minutes. The mixture was then transferred to a separatory funnel, wherein the ethanol and hexane phases were allowed to separate (20 min.). Hexane and ethanol phases were separately collected. The ethanol phase was then back washed with 6 volumes hexane, shaken and allowed to separate. The hexane phase was back washed with 4 volumes 95% EtOH and the phases allowed to separate for 1 hour. If extra hexane was needed to facilitate the separation, it was added. The collected ethanol and hexane phases seemed to consist of only one phase. However, upon storage of each phase at refrigerator temperature, (i.e. 4° C), each of the collected phases was separated into two new ethanol and hexane phases which were again collected separately.

The total hexane phase is then subjected to rotary evaporation (under vacuum, 37° c) until a translucent hexane extract of chloroform/methanol fraction is obtained (HxCMFr). The recovery of this phase is about 82±3% of CMFr.

Other organic solvents can be used to extract omentum and the invention is not limited to the specific solvents mentioned above.

(2c) Preparation of Omentum Lipid Extract By Supercritical Gas Extraction

We also note the use of supercritical gas extraction for omentum fractions and factors as described infra and in our co-pending U.S. Patent Application Serial No. 793,622, filed October 31, 1985 and entitled "Supercritical Fluid Extraction of Animal Derived Material" by Kamarei and hereby incorporated by reference.

In this process a supercritical (SC) fluid (F) such as SC-$CO_2$ is preferably used to extract omentum. SCF has increased solvation power at temperatures above the critical pressure (Pc) and critical temperature (Tc).

Polar materials such as gangliosides remain in the residue while the extract contains the more non-polar or lipid materials such as triglycerides. Temperatures used for example are 38-39° C and extractor pressure is about 3500 psi. Thus these conditions can avoid extraction using toxic materials, or inefficient extraction or use of expensive and time-consuming extractions and materials.

The following example describes the use of supercritical gas extraction for porcine homogenized omentum and porcine omentum CMFr.

APPARATUS

A process development unit (PDU) is used. Briefly, the PDU consists of an extractor and three separators, which are housed in an oven at a predetermined temperature. A supercritical solvent in this case, $CO_2$, is pumped into the extractor and then flows, sequentially, through separators, and the "knock out" vessel. Pressure is maintained by back pressure regulators. Gas, e.g., $CO_2$, at approximately normal atmospheric pressure, exits the vent, when the extraction is completed.

2(d) Method for Supercritical Gas Extraction of Porcine Omentum

Omentum samples were melted in a separate, nitrogen purged oven at about 40° C, and then transferred into the extractor.

The vessels were purged with low pressure $CO_2$, and were then brought to the temperatures and pressures indicated in Tables 2-3. $CO_2$ was then pumped at a rate of about 0.3 lb/min through the system until a weight of about 200 times the sample weight was pumped. Pressure was bled, and samples in each of the vessels and extractor were removed, weighed, and analyzed.

In these experiments, it was observed that close to, and above the critical point of the $CO_2$ used, dissolving power increased with an increase in temperature, at constant density and with increased density at constant temperature. For example, a portion of the sample, dissolved in the extractor at 3500 psi, precipitates out at l500 psi in the first separator vessels. Further reductions in pressure/density cause additional fractions to precipitate out, until, at ambient pressure, the supercritical gas contains no dissolved material.

The residues obtained from the extractor were found to be insoluble in $CO_2$. Polar portions of the material, such as gangliosides, were expected to remain in the fraction, while extract fractions were expected to be rich in neutral, non-polar components. This has been observed to be the case, as the insoluble residue is found to contain polar materials, such as gangliosides, while non-polar materials, such as triglycerides, are found in the extracts.

While a single extractor, 3 separators and collecting vessels, and l knock out vessel are used in this embodiment, one skilled in the art will recognize that the number and combinations of each of these is a matter of design choice.

TABLE 2

Material: Porcine Adipose Tissue CMFr Extract

CONDITIONS

Sample Weight Charged: 106.5 gm
Supercritical Solvent: $CO_2$
Solvent Recirculated: 46.9 lbs.
Solvent to Feed Ratio: 200/1
Solvent Flow Rate: 0.3 lb./min.

| | Extractor | Separator #1 | Separator #2 | Separator #3 | V5 |
|---|---|---|---|---|---|
| Temperature ($^0C$) | 38-39 | 40 | 37 | 33 | — |
| Pressure (psi) | 3500 | 1500 | 1300 | 1100 | 5 |
| Density | 0.87 | 0.69 | 0.62 | 0.26 | |

MATERIAL BALANCE

Total Recovered (grams): 81.6
% Recovery: 76.6

| | V9 | V8 | V7 | *V6 | *V5 |
|---|---|---|---|---|---|
| Weight (grams) | 11.4 | 58.5 | 10.0 | 0.7 | 1.0 |
| Weight (% of feed) | 10.7 | 54.9 | 9.4 | 0.7 | 0.9 |

*Comments: V9 very viscous, off-white color

*Washed from vessel with Hexane, evaporated off but may be residual

TABLE 3

Material: Porcine Homogenized Omentum

CONDITIONS

| | |
|---|---|
| Sample Weight Charged: | 109.6 gm |
| Supercritical Solvent: | $CO_2$ |
| Solvent Recirculated: | 48.3 lbs. |
| Solvent to Feed Ratio: | 200/1 |
| Solvent Flow Rate: | 0.3 lb./min. |

| | Extractor | Separator #1 | Separator #2 | Separator #3 | V5 |
|---|---|---|---|---|---|
| Temperature ($^0C$) | 38-39 | 40 | 37 | 34 | -- |
| Pressure (psi) | 3500 | 1500 | 1300 | 1100 | 5 |
| Density (gm/cc.) | 0.87 | 0.69 | 0.62 | 0.26 | |

MATERIAL BALANCE

| | |
|---|---|
| Total Recovered (grams): | 82.3 |
| % Recovery: | 75.1 |

| | V9 | V8 | V7 | *V6 | *V5 |
|---|---|---|---|---|---|
| Weight (grams) | 36.7 solids | 38.6 | 1.2? | 4.1 | 1.7 |
| Weight (% of feed) | 33.5 | 35.2 | 1.1 | 3.7 | 1.6 |

*Comments: V9 solids tissue-like
V8 clean white solid melted @ 45°C

*Washed from vessel with hexane, evaporated off but may be residual

(2e) Use of Detergents for Lipid Isolation

Lipids are displaced from homogenized cell membranes, or other complexes involving proteins, by amphipathic detergent molecules which render the proteins "soluble" in aqueous media. The released lipid material is recovered by flotation after centrifugation as disclosed in serial no. 811,375 filed on December 20, 1985, herein incorporated by reference.

It is interesting to note that when omentum "powder" (rather than traditional cell breaking method) is used, not only processing and handling of the raw material is much easier for all types of follow-up extractions, but the percent lipid recovery of all above examples is higher than the traditional method of cell breaking known in the art.

(3a) Preparation of the Total Lipid Extract from Feline Omentum

The method of Goldsmith et al. J. Amer. Med. Assoc., 252: 2034-36 (l984) was used. Also see co-pending U.S. Application S.N. 642,624 filed August 20, 1984 entitled "Angiogenic Factor and Method for Producing Angiogenesis" by Catsimpoolas, et al., both of the above references being hereby incorporated by reference. In another co-pending application U.S. Serial No. 782,724, filed on October 1, l985 entitled "Compositions Containing Lipid Molecules with Enhanced Angiogenic Activity" by Catsimpoolas et al., hereby incorporated by reference, various omentum extracts are analyzed and their components described. Supercritical gas extraction can also be used to extract omentum lipids as described in copending U.S. Application 793,622, filed October 31, 1985 entitled "Supercritical Fluid extraction of Animal Derived Material" by Kamerei.

Various samples which also could be used in the composition formulation may be prepared as follows:

Reza Sample #1.

400 grams of porcine neutral lipids (HxCMFr, batch #35 and 36-85-P.O.) from omentum powder. This source is obtained by initial extraction which chloroform/methanol followed by removal of polar lipids, via ethanol extraction, form the extract.

Reza Sample #2)

300 grams of porcine neutral lipids (HxFr, batch #37-85-P.O.) from omentum powder. This source is obtained directly by hexane extraction of omentum powder.

Reza Sample #3)

300 grams of SuperCritical-$CO_2$ extract of porcine omentum powder (equal portions of V8 and V9 extracts of the last 3 runs).

Reza Sample #4)

300 grams of Angio-Medical porcine subcutaneous adipose tissue i.e. P.S.A.T. (batch #38-85). This source is directly extracted by hexane (HxFr).

Reza Sample #5)

50 grams of Angio-Medical P.S.A.T. (batch #15-85-F.B.). This source which should be similar to sample #4. is extracted by SuperCritical $CO_2$ (V8).

Reza Sample #6

500 grams of thermally extracted porcine omentum powder (batch #39-85-P.O.).

Formulations of the Omexin™ Hair Lotion

The hair growth composition is manufactured under the trademark Omexin™ for a hair lotion owned by Angio-Medical Corporation. The composition may be formulated in any conventional topical form such as lotions, creams, ointments, aerosols, sticks, gels, and other forms known in the art. Preferably, a lotion is formulated having a percentage by weight of mammalian omentum material ranging from about 5% to 20% of the topical preparation, preferably from about 14-18%.

The following examples describe the manner and process of making and using the lotion composition and set forth the best mode contemplated by the inventor of carrying out the inventive method, but are not to be construed as limiting.

## EXAMPLE 1

### Omexin™ Hair Lotion

| CTFA NAME | TRADE NAME | SUPPLIER* | AMOUNT (%) |
|---|---|---|---|
| Omentum (Porcine HxFr) | | Angio-Medical | 15.00 |
| Sorbitan Stearate | Span 60 | ICI | 3.05 |
| Propylparaben | Propylparaben | Mallinckrodt | 0.10 |
| Polysorbate 60 | Tween 60 | ICI | 2.2 |
| Glyceryl Stearate | Myverol 18-07 | Eastman Kodak | 1.13 |
| Tocopheryl Acetate | Vitamin E Acetate | Eisai USA | 0.10 |
| Dimethicone | Dow Corning 200,. 350 cps | Dow Corning | 3.0 |
| Carbomer 940 (2% soln.) | Carbopol 940 (2% soln.) | B.F. Goodrich | 5.0 |
| Water | Purified Water USP | | 49.42 |
| Butylene Glycol | 1,3-Butylene Glycol | Union Carbide | 5.00 |
| Methylparaben | Methylparaben | Mallinckrodt | 0.30 |
| Trisodium EDTA | Trisodium EDTA | Dow | 0.10 |
| Phenoxyethanol | Emeressence 1160 | Emery | 0.50 |
| Triethanolamine | Triethanolamine | Union Carbide | .10 |
| Sodium Hyaluronate (1% soln.) | Actimoist® (1% soln.) | Active Organics | 15.00 |
| | | | 100.00 |

*Mallinkrodt-Chemical Co., P.O. Box 5439 St. Louis, Mo 63147
ICI/Concord Pike/New Murphy Rd. Wilmington DC 19817
Eastman Kodak/343 State ST,/Rochester NY 14650
Eisai USA Inc./Los Angeles, CA
Dow Corning/P.O. Box 1767/Midland, MI 48640
BF Goodrich/6100 Oaktree Blvd/Cleveland, Ohio 44131
Union Carbide/Old Ridgebury Rd/Danbury, CT 06817
Dow Chemical Co/2020 Dow Centre/Midland Mi 48640
Emery/1300 Carew Tower/Cinncinati Ohio 45202
Active Organics/7715 Densmore Ave/Van Nuys Calif 91406

## PREFERRED INGREDIENTS for Omexin™ HAIR LOTION

### Sodium Hyaluronate

This ingredient is sold under the trademark ACTIMOIST® owned by Active Organics of Van Nuys, California.

The preferred sodium hyaluronate used in the manufacturing of the hair growth composition has the following chemical analysis:

I. molecular weight - not less than 2,500,000;
2. sodium hyaluronate content - not less than 95%;
3. protein - not more than 0.2%;

4. nucleic acid - not more than 0.2;
5. chondroitin sulfate - not detected;
6. moisture content - not more than 6.0%;
7. color and clarity of solution - colorless and transparent.

MANUFACTURE PROCEDURE FOR OMEXINTM HAIR LOTION

Prior to the manufacturing of the lotion, a carbomer 940 solution which is 2% by weight of the composition formula is prepared. To prepare the solution the following ingredients are needed.

| Ingredients | Amount (%) |
| --- | --- |
| Purified Water | 97.7 |
| Methylparaben | 0.20 |
| Propylparaben | 0.10 |
| Carbomer 940 | 2.0 |

Porcine omentum extract prepared by brittle grinding and direct hexane extraction was used in the preparation of this lotion.

To manufacture OmexinTM hair lotion, purified water is added to a main steam-jacketed stainless steel kettle, preferably equipped with an anchor mixer and preferably heated to 80-85° C. Butylene glycol is then added and mixed. Methylparaben and Trisodium EDTA are sprinkled into the batch and the ingredients are continuously mixed until they are in solution. At that point Carbomer 940 (2% solution) is added. The temperature is maintained at 75° C and the kettle is covered to avoid evaporation.

In an auxiliary steam-jacketed stainless steel kettle equiped with a propeller mixer, the ingredients of the oil phase are added as follows: Omentum, sorbitan stearate, propylparaben, polysorbate 60, glyceryl stearate, and Dow Corning 200 Fluid. The mixture is heated to preferably 70° C while mixing to achieve uniformity of solution without overheating the mixture.

The oil phase is then added into the main kettle while mixing. Temperatures should be adjusted to 75° C. triethanolamine, 99% and phenoxyethanol are added into the combined mixture and the hot water from the jacketed kettle is drained to begin cooling the batch.

The batch is continually mixed and cooled and at approximately 45° C, sodium hyaluronate is added.

When the mixture has cooled to 35° C, Dow Corning 200 fluid is added. The mixture should be continually mixed and cooled down to approximately 30° C.

At the preferred temperature, remove the batch from the kettle by either gravity or with the aid of a suitable pump into a clean sanitized container. One should avoid incorporating air into the batch while pumping. Once the mixture is contained, the container should be closed tightly to prevent evaporation.

Procedure for Use

To use the resulting inventive lotion, the OmexinTM hair lotion should be applied to bald and balding areas of the scalp twice daily, preferably in the morning and at night, to a dry scalp and gently rubbed for approximately ten seconds.

The OmexinTM hair lotion should not be applied immediately before shampooing and a mild shampoo should be used at the frequency normally employed by the user.

Hair Growth Study of OmexinTM Hair Lotions

Protocol

19 male subjects ages 18-49 with androgenetic alopecia (Hamilton types IV-V) were treated topically with Omexin™ hair lotion for 8 months.

The subjects selected had a medical history and physical examination performed to rule out exclusionary conditions, such as heart disease, kidney disease, etc.

Hair growth was evaluated at monthly intervals. Further, safety examinations, including temperature, pulse, respiratory rate, blood pressure, weight, general skin exam, palpitation of lymph nodes, pulmonary and cardiac auscultation were performed at each evaluation visit. Additionally, retinal vasculature and possible development of corneal vasculature were assessed to exclude any possibility of systemic angiogenic effects.

Experimental Method

19 individuals completed the eight (8) months of study reported in this application. Only subjects with dark hair were selected for this study, since blonde hair is harder to count. Further, there were no female subjects selected or any subjects having cardiac disease, hypertension, hematological disease or any other ailments.

Omexin™ hair lotion was supplied by Angio-Medical Corporation of New York, New York.

Dosage and Administration

Applications of the Omexin™ hair lotion were placed on the entire top portion (i.e., frontal/crown/vertex) of the scalp, excluding the occipital and lower parietal hair bearing areas. Dosages of 1 cc. for each patient were dispensed on the areas and spread with the fingers for uniform coverage.

Application occured twice daily, once in the morning and once at night, at approximately 12 hour intervals and to a dry scalp. A mild shampoo was used by the subjects to shampoo their hair at a frequency normally employed by the subjects.

No Evidence of Systemic Angiogenesis

In a study to determine if there is any possible systemic angiogenesis with topical Omexin™ hair lotion 18 men, ages 18-49 were treated topically with the lotion for 9 months. Serial ophthalmologic exams showed no changes in retinal vasculature in the subjects according to Aurthur P. Bertolino, M.D., Ph.D of the New York University Medical Center. Thus, the likelihood of a systemic effect from the topical application of omentum material appears unlikely.

Evaluation of Hair Growth

Several techniques for evaluating hair growth were employed, including color photography using standardized distance and lighting of the selected scalp area before treatment began and at each monthly evaluation. Other evaluation techniques included skin biopsy, visual gross estimation of new hair growth by both the physician and the subject and a hair count per defined area.

Two weeks before the study began, a site at the left margin of the area of vertex alopecia was chosen on each subject. A four millimeter punch biopsy was performed at the site medial to the left lateral margin of vertex alopecia. Closure of the biopsy was achieved with one simple monofilament suture. Two weeks following the punch biopsy, the suture was removed and application of study medications was commenced. Biopsies will be repeated at the end of the one year study.

Biopsy specimens were sectioned horizontally according to the method of Headington, J.G., Arch. Derm. 120: 449-456 (1984). By this means an accurate hair count was achieved. Special staining for blood vessels were also performed to assess angiogenic effects.

To evaluate the base line characteristic of each subject, dimensions of areas of baldness or alopecia were measured or recorded. Photographs were taken using a 35 millimeter camera with a 100 millimeter macrolens and Kodachrome® 25 slide film. Subjects were seated and the scalp photographed from above and from a frontal view. Hair counts were performed in a 2 centimeter circle using the biopsy scar as the center of the circle. These counts were repeated monthly during the study.

Hair counts involved separate enumeration of terminal, indeterminate and vellus hairs. Hair counts were performed by the same investigator throughout the study to avoid variation.

Visual gross estimation by the investigator of new hair growth was graded as follows:

W = worsening of alopecia;
0 = no new hair growth;
1 = new growth of vellus hairs;
2 = minimal new growth of terminal hairs;
3 = moderate new growth of terminal hairs;
and
4 = dense new growth of terminal hairs.

Subjective evaluation by the subject regarding his hair growth was graded as follows;

W = worsening of hair loss;
0 = no new hair growth;
1 = minimal new hair growth;
2 = moderate hair growth; and
3 = dense new hair growth.

Hair loss was evaluated by the subjects during the first five months of using the OmexinTM hair lotion. Hair loss was graded at base line as minimal, moderate or marked and at each of the monthly visits was evaluated as:

I = increased hair loss (since the preceding visit;
0 = no change in hair loss (compared to base line);
D = decreased hair loss (since prior visit); and
S = stabilized at a reduced rate of hair loss.

Results of the Study

Of the 19 subjects only two subjects did not have total new hair growth (i.e. vellus, indeterminate and terminal) as graphically shown in Fig. 9. A decrease in hair loss was observed in about 20% of the subjects. Eight subjects had a decrease in the diameter of the bald spot and six subjects showed no change in its size.

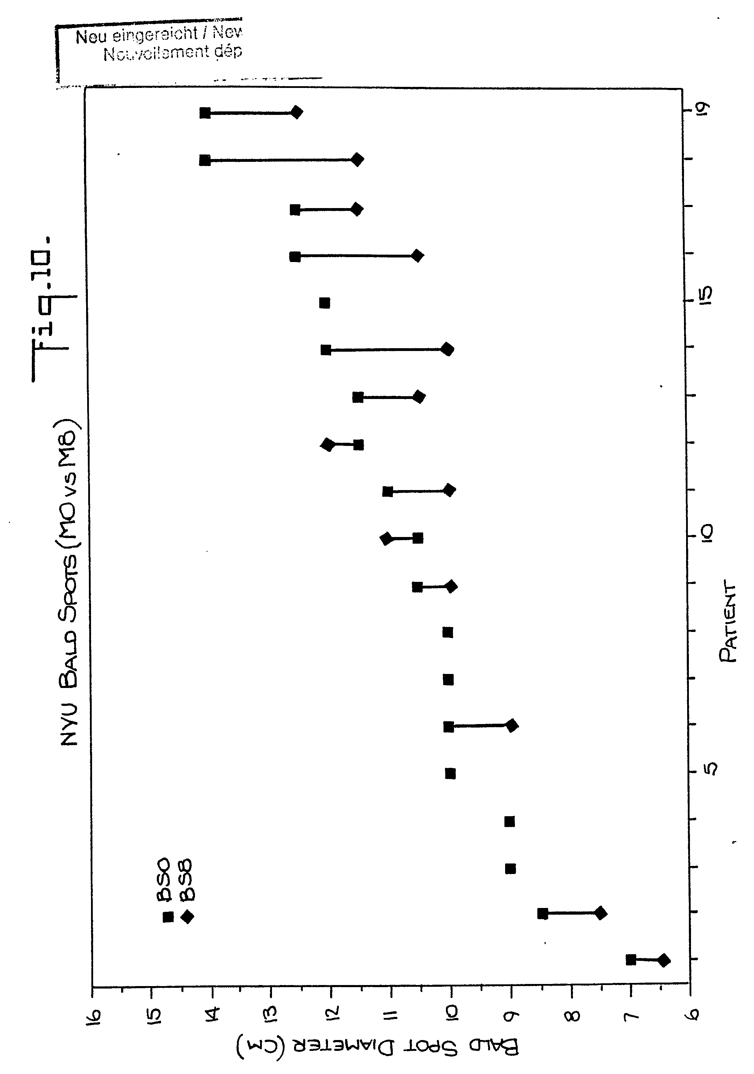

Comparison photographs of two subjects are included as Figures 3-6. Prior to beginning treatment photographs of the subjects' heads were taken (Figs. 3 and 5) and six months after treatment, comparison photographs (Figs. 4 and 6) illustrate the increase in terminal hair growth.

Evaluation of Study Results

Comparison to Minoxidil

As described in the Background of the Invention, the following six articles reporting hair regrowth results using 2% Minoxidil were used to compute the SinnTM plot predictor illustrated in Figure 7:

Shupack et al., J. of the Amer. Acad. of Derm. I6: 673-6 (I987);
de Villez, R. L., Arch. Dermatol. 121: 197-202 (1985);
de Villez, R., J. of the Amer. Acad. of Derm. I6: 669-72 (I987);
Rietschel et al., J. of the Amer. Acad. of Derm. I6: 677-85 (I987);
Olsen et al., J. of the Amer. Acad. of Derm. I3: I85-I92 (I985); and
Olsen et al., J. of the Amer. Acad. of Derm. 15: 30-37 (I986).

From the data presented in these six references, Table 5 was compiled as follows:

Table 5

2% Minoxidil b.i.d.

| | | | Shupack '87 | Devillez ' 87,'85 | Reitsechel '87 | Oslen '86 | Oslen '85 |
|---|---|---|---|---|---|---|---|
| Baseline | 1. | Hamilton | III,IV | | | III(12),IV(7) | III(9), IV(15) V(16) VI (1) |
| | 2. | Bald Spot, (cm) | 5-10 | 2.5 | | 3.7 | |
| | 3. | M [Bald Spot (cm)] | 7.5 | | | | |
| | 4. | Age (years) | 24-49 | | | | |
| | 5. | M [Age (years)] | 32,(29) | | | 35 | |
| | 6. | Baldness Duration | 8,(6) | | | 9.1,(2-20) | 9.4,(2-18) |
| | 7. | Spot Size | 1" | 1" | | 1" | 1" |
| | 8. | M [Tot] | | | 63.5 | 528,(310-694) | |
| | 9. | M [T] | | | 46 | | 126 |
| | 10. | M [I] | | | 4 | | 61 |
| | 11. | M[T+I] | 93 | | 50 | 365,(142-566) | 187 |
| | 12. | M[V] | | | 14 | 163(0,410) | 95 |

M = Median
Tot = total hairs
T = terminal
I = indeterminate
V = vellus
N = % Cummulative median change

The data presented in Table 5 was graphed to assess growth of terminal and indeterminate hairs over the period of the study, and as illustrated in Figure 8 Rietschel, supra reported the least increase of new hair growth and Olsen, supra (l985) reported the greatest increase in new hair growth.

The inventors then extrapolated the data from Olsen, supra (l985) for the increase in terminal hairs based on the baseline number of indeterminate hairs for l2 months to produce the SinnTM plot predictor illustrated in Figure 7.

Results obtained from the five month evaluation of the subject invention were then compiled and graphed for the OmexinTM hair lotion treatment as shown in Figure 9.

Further, the experimental data were evaluated and plotted on the graph shown in Figure 7. As illustrated, at the end of three months, the number of terminal hairs produced by the treatment with the OmexinTM hair lotion equalled the number of terminal hairs achieved by the 2% Minoxidil lotion after five months of application.

Initial results show that the OmexinTM hair lotion increases the number of terminal hairs in the first four months of application over the number predicted by the 2% Minoxidil solution. As illustrated in Figure 7 the average terminal hair growth achieved with OmexinTM hair lotion treatment after four months equalled the average hair growth predicted from use of 2% Minoxidil after five months. Furthermore, after eight months of treatment with OmexinTM hair lotion, the average hair growth achieved exceeded the hair growth predicted from use of Minoxidil after nine months. Thus treatment with the invention composition results in a greater increase of hair growth earlier in the treatment period as compared to the 2% Minoxidil treatment.

A statistically significant decrease in the diameter of the bald spots of the subjects was also observed in the 19 subjects as illustrated in Figure 10.

## EXAMPLE II

A preferred cream formulation known as OmexonTM hair Cream is as follows:

CREAM INGREDIENTS (OmexonTM hair Cream)

| CTFA NAME | TRADE NAME | SUPPLIER* | AMOUNT (%) |
|---|---|---|---|
| Omentum (Porcine HxFr) | | Angio-Medical | 25.00 |
| Sorbitan Stearate | Span 60 | ICI | 3.60 |
| Propylparaben | Propylparaben | Mallinckrodt | 0.10 |
| Polysorbate 60 | Tween 60 | ICI | 2.5 |
| Glyceryl Stearate | Myverol 18-07 | Eastman Kodak | 2.15 |
| Tocopheryl Acetate | Vitamin E Acetate | Eisai USA | 0.10 |
| Dimethicone | Dow Corning 200, 350 cps | Dow Corning | 5.00 |
| Carbomer 940 (2% soln.) | Carbopol 940 (2% soln.) | B.F. Goodrich | 12.50 |
| Water | Purified Water USP | | 13.60 |
| Butylene Glycol | 1,3-Butylene Glycol | Union Carbide | 5.00 |
| Methylparaben | Methylparaben | Mallinckrodt | 0.30 |
| Trisodium EDTA | Trisodium EDTA | Dow | 0.10 |
| Phenoxyethanol | Emeressence 1160 | Emery | 0.50 |
| Triethanolamine | Triethanolamine | Union Carbide | .25 |
| Sodium Hyaluronate | Actimoist® | Active Organics | 25.00 |
| Cetyl alcohol | Cetyl alcohol | | .25 |
| Stearic acid | Stearic acid | | 3.0 |
| | | | 100.00 |

*Mallinkrodt-Chemical Co., P.O. Box 5439 St. Louis, Mo 63147
ICI/Concord Pike/New Murphy Rd. Wilmington DC 19817
Eastman Kodak/343 State ST,/Rochester NY 14650 Eisai USA/Los
Dow Corning/P.O. Box 1767/Midland, MI 48640
BF Goodrich/6100 Oaktree Blvd/Cleveland, Ohio 44131
Union Carbide/Old Ridgebury Rd/Danbury, CT 06817
Dow Chemical Co/2020 Dow Centre/Midland Mi 48640
Emery/1300 Carew Tower/Cinncinati Ohio 45202
Active Organics/7715 Densmore Ave/Van Nuys Calif 91406

This formula variation is manufactured by the process described supra for the lotion formulation. Other formulations of the cream varying the proportions in the cream and achieving a nonseparating cream at 50 °C will be obvious to those skilled in the art.

Irritability, Mutagenicity and Toxicological Studies on OmexinTM Composition

Irritability, mutagenicity and toxicological studies were performed as described infra. Similarly test methods were described previously in copending U.S. Patent Application, Serial No. 811,505, filed on December 20, 1985 entitled "Lipids from Omentum and Methods for Cosmetic Use" by Catsimpoolas, et al.; herein incorporated by reference.

The Ames mutagenicity test (Ames et al., (1975) Mutation Research 31: 347-364) was performed on the porcine omentum extract (POCMFr) by Product Safety Laboratories of 725 Cranbury Rd, East Brunswick, New Jersey 08816. The mutagenic potential of the test material was measured by its ability to induce reverse ("back") mutations in specially constructed strains of the bacterium Salmonella Typhimuruim. Mutagens which require activation by enzymes found in mammalian tissues are detected in the bacterial assay by the addition of mammalian microsomes (S-9 fractions).

Conclusion:

Positive and negative controls were used for all tests or strains. There was no evidence of any irritability, mutagenicity or toxicological effects of the OmexinTM hair lotion or cream from these studies.

Positive Controls:

In the absence of metabolic activation, N-Methyl-N-nitro-N-nitrosoguanidine (MNNG), 9-aminoacridine (9AA), and 2-nitrofluorene (2NF) were used with the appropriate strains. In the presence of metabolic activation, 2-amino-anthracene (2AA) was used as a positive control for all tester strains.

Negative Controls:

The spontaneous mutability of each strain in the presence and absence of metabolic activation was determined. Solvent controls were used at the highest volume tested in the experiment.

The Salmonella/mammalian microsome assay of Ames (Ames et al., 1975) utilizes specially constructed strains of the bacterium, Salmonella typhimurium, to detect mutagenic activity of chemicals. The rationale for the test lies in the established correlation between mutagenic activity and carcinogenic potential, and, with birth defects and heritable disease.

Gene (point) mutations are usually recognized as change in a phenotypic characteristic. In the Ames assay, the dependence of the Salmonella tester strains on an exogenous source of histidine (auxotrophy) is altered by reverse mutation to histidine independence (prototrophy). In this particular system, the reversion occurs by either base-pair substitution or frameshift mutation, depending upon the strain. The various strains carry additional genetic markers which enhance their sensitivity to mutagens.

In the assay, tester strains are exposed to a series of doses of the test article at concentrations up to the highest practicable doses within the limitations of toxicity or solubility. Mutagenic activity is manifested by an increase in the numbers of mutant colonies which grow in the absence of histidine, compared to the spontaneous (background) number of mutants. Mutagens which require activation by enzymes found in mammalian tissues, are detected in the bacterial assay by the addition of mammalian microsomes (S-9 fractions).

Ames Test Materials:

Bacterial Indicator Strains

Salmonella typhimurium strains TA 1535, TA 1537, TA 1538, TA 98 and TA 100 were originally obtained from Dr. Bruce N. Ames, Berkeley, California. Strains were propagated in nutrient broth containing 0.5% NaCl, checked for appropriate genetic markers, and stored frozen (Revco freezer) after addition of DMSO (dimethyl sulfoxide) to approximately 8% final concentration.

Media

1. Mutrient broth (Difco) was supplemented with 0.5% NaCl.
2. Top agar contained 0.6% Difco agar, 0.5% Nac1, 0.5 mM biotin, and 0.5 mM L-histidine-HC1.
3. Minimal-glucose agar medium contained 1.5% Bacto-Difco agar in Vogel-Bonner Medium E (Vogel and Bonner, 1956) with 2% glucose.
4. Tryptone agar.

Positive Control Chemicals

N-methyl-N-nitro-N-nitrosoguanidine (MNNG), 9-aminoacridine (9-AA), and 2-nitrofluorene (2-NF) were used in the direct mutagenesis assay with indicator strains as shown in Appendix 1.

2-aminoanthracene (2-AA) was used with all strains in the assay with metabolic activation as shown in Appendix 2.

Solvent

Acetone was the solvent used for the test article.

1. S-9 Metabolic Activation Mixture

S-9 was thawed on the day of assay, diluted first in Tris-KCl buffer to a protein concentration of 10 mg ml, then added to S-9 buffer to give a final concentration of 100 microliter S-9/ml. The complete S-9 mix was passed through a 0.45 um disposable Nalgene filter unit for sterilization and stored at approximately 4° C until use on the same day.

The following components and their final concentrations were used in S-9 buffer:

| Component | Final Concentration/ml S-9 mix |
|---|---|
| $MgCl_2$ | 8 micromole |
| KCl | 33 micromole |

| Component | Final Concentration/ml S-9 mix |
|---|---|
| Glucose-6-phosphate | 5 micromole |
| NADP | 4 micromole |
| $NaH_2PO_4 \cdot H_2O$, pH 7.4 | 100 micromole |

Methods for Ames Test:

Bacterial Preparation

The cultures used in this assay were started from the frozen stock cultures in 21 ml of nutrient broth with 0.5% NaCl and incubated 12-16 hours on a shaker water bath at 37 °C. The fresh cultures were stored in a refrigerator while tests for the appropriate genetic markers were conducted. These tests, which were carried out as described by Ames et al. (1975), showed no growth in the absence of histidine, and no growth in the presence of crystal violet or after exposure of ultraviolet light. Strains TA 1535, TA 1537 and TA 1538 were susceptible to ampicillin, while strains Ta 98 and TA 100 were resistant.

## Preliminary Toxicity Testing

The effect of the test article on the survival of the bacterial strains was determined prior to the Ames bioassay. This was accomplished by adding 0.l ml of different levels of the test article and solvent to tubes containing 2.0 ml top agar (at 45 °C) and 0.1 ml of the tester strain. After mixing, the tube contents were poured onto the surface of tryptone agar plates. The plates were incubated at 37 °C for 40-48 hours and the background lawn of bacteria in test article plates was compared to the bacterial lawn in the solvent plates. Toxicity on the tryptone agar plates was detectable by a thinning or disappearance of this background lawn of bacteria.

## Plate Assay for the Detection of Direct Acting Mutagens

Assays were performed according to the method of Ames et. al. (1975). The test material was dissolved and diluted in solvent (acetone) on the day of the assay. The following were added, in order, to 2 ml of molten top agar (45 °C):

1. 0.1 ml of culture of the appropriate indicator strain;
2. 0.1 ml of the appropriate concentrations of test material, control article, or solvent.

Each tube was prepared individually, immediately vortexed, and the contents poured over the surface of a minimal-glucose agar plate. Each plate was rotated to evenly distribute the top agar before it hardened. All plates were incubated for approximately 48 hours at 37 °C. After incubation, the plates were observed for revertant colonies and the colonies were counted and recorded.

Five doses of test article were tested in triplicate with each strain. Positive control articles for each strain and spontaneous revertant controls consisting of the indicator strains and solvent were run concurrently. The solvent and the highest dose level of the test material were checked for sterility by adding 0.1 ml of each to 2 ml of top agar without the test organisms and pouring the entire contents over minimal-glucose agar plates.

## Plate Assay for the Detection of Mutagens Requiring Metabolic Activation

Plate assays with S-9 were conducted as described above, the only modification being the addition of 0.5 ml of S-9 mix to each tube of top agar immediately before vortexing and pouring.

The positive control article for each strain and spontaneous revertant controls consisting of the indicator strains and S-9 mix were run concurrently. The S-9 mix was checked for sterility by adding 0.5 ml to 2 ml of top agar without the test organisms and pouring the entire contents over minimal-glucose agar plates. All plates were incubated approximately 48 hours at 37 °C.

## Criteria

The following criteria were used in the evaluation and reporting of the mutagenic potential of the test material:

1. The spontaneous revertant levels for each strain when used in either the direct plate assay or the activated plate assay must not differ significantly from the range of historical values shown in Appendix 3.
2. All sterility controls must be negative.
3. All positive controls must demonstrate that the indicator strains are functional with known mutagens as evidenced by an increase of at least three times the number of revertant colonies per plate as the spontaneous revertant controls.

4. To be considered positive for mutagenic activity, the test material should exhibit a dose response effect (increasing number of revertant colonies with increased amounts of the test sample).

5. To be considered mutagenic for strains TA 1535, TA 1537, TA 1537, TA 1538, and TA 98, the test sample should produce a positive dose response over three concentrations with the lowest increase in revertants/plate greater than or equal to 3x the solvent control value or the S-9 fraction control value, as applicable.

6. To be considered mutagenic strain TA 100, the test sample should produce a positive dose response over three concentrations with at least one dose producing an increase in revertants/plate greater than or equal to 3.5 x the solvent control value or the S-9 fraction control value, as applicable.

A valid test requires that criteria 1, 2 and 3 be met. To be considered mutagenic, criteria 4, and 5 or 6 must also be met.

AMES TEST

Appendix I

POSITIVE CONTROL CHEMICALS IN THE DIRECT MUTAGENESIS ASSAY

| Indicator Strain | Positive Control Chemical | Conc. (ug/plate) | Solvent | Mutagenic Activity |
|---|---|---|---|---|
| TA 1535 | MNNG | 5.0 | DMSO | Base-pair Substitution |
| TA 1537 | 9-AA | 250.0 | DMSO | Frameshift Mutation |
| TA 1538 | 2-NF | 0.5 | DMSO | Frameshift Mutation |
| TA 98 | 2-NF | 0.5 | DMSO | Frameshift Mutation |
| TA 100 | MNNG | 5.0 | DMSO | Base-pair Substitution |

MMNG, N-methyl-N-nitro-N-nitrosoguanidine; 9-AA, 9-aminoacridine; 2-NF, 2-nitrofluorene; DMSO, dimethylsulfoxide.

AMES TEST

Appendix 2

POSITIVE CONTROL CHEMICALS IN THE
METABOLIC ACTIVATION MUTAGENESIS ASSAY

| Indicator Strain | Positive Control Chemical | Conc. (ug/plate) | Solvent | Mutagenic Activity |
|---|---|---|---|---|
| TA 1535 | 2-AA | 2.5 | DMSO | Base-pair Substitution |
| TA 1537 | 2-AA | 2.5 | DMSO | Frameshift Mutation |
| TA 1538 | 2-AA | 2.5 | DMSO | Frameshift Mutation |
| TA 98 | 2-AA | 2.5 | DMSO | Frameshift Mutation |
| TA 100 | 2-AA | 2.5 | DMSO | Base-pair Substitution |

2-AA, 2-aminoanthracene; DMSO, dimethylsulfoxide.

AMES TEST

Appendix 3

HISTORICAL BACKGROUND OF SPONTANEOUS REVERTANT LEVELS FOR THE BACTERIAL INDICATOR STRAINS*

| Strain | Mean (# Revertants/Plate) | Standard Deviation | Range (# Revertants/Plates) |
|---|---|---|---|
| A. Plate Assay Without Metabolic Activation System | | | |
| TA 1535 | 18.09 | 8.04 | 2-43 |
| TA 1537 | 6.29 | 2.47 | 2-15 |
| TA 1538 | 13.72 | 4.48 | 5-32 |
| TA 98 | 23.54 | 9.03 | 9-60 |
| TA 100 | 144.94 | 41.89 | 66-318 |
| B. Plate Assay With Metabolic Activation System | | | |
| TA 1535 | 15.86 | 5.98 | 5-40 |
| TA 1537 | 8.19 | 2.68 | 2-15 |
| TA 1538 | 26.27 | 10.22 | 12-65 |
| TA 98 | 39.85 | 14.31 | 16-90 |
| TA 100 | 156.53 | 44.61 | 73-407 |

## Toxicity Studies

Oral toxicity tests on the the PO HxFr (white creme) were done by Product Safety Labs, 340 Commercial Avenue, New Brunswick, NJ 08901. The test material was warmed under tap water until liquified.

During the test period the animals (rats) were each uniquely identified and individually housed in stainless steel wire bottomed cages in an environmentally controlled room with a l2 hour light/dark cycle. Feed and water were provided freely after dosing.

### Procedure

Acute Oral Toxicity. FHSLA, I6 CFR I500.3. The rats were fasted for I8 hours and then individually and singly dosed by gavage with 5.0 g/kg body weight of test material. The rats were individually caged and observed for mortality or other signs of gross toxicity for I4 days. Feed and water were provided freely.

The oral $LD_{50}$ of the Porcine HxFr test material is greater than 5.0 g/kg.

At autopsy, no macroscopic changes could be noted in any of the internal organs of the treated rats and the test material is not toxic as defined in I6 CFR I500.3.

## Eye Irritation Studies

Eye irritation studies were conducted on the Omexin™ hair lotion on rabbits by Leberco Testing Inc. of Roselle Park, N.J.

During the test period six healthy animals were individually housed in wire bottomed cages in an environmentally controlled room. Feed and water were provided freely after dosing.

Each animal had 0.1 ml of the Omexin™ hair lotion instilled into the conjunctival sac of the right eye. The untreated left eye of each animal served as a control for that animal.

The treated and untreated eyes were examined and graded after 24, 48 and 72 hours post installation and the readings obtained are shown in Table 6 infra.

TABLE 6

EYE IRRITATION OBSERVATIONS

| RABBIT NO. | HOURS POST INSTILLATION | RESULTS (+ OR –) I | II | III | IV |
|---|---|---|---|---|---|
| 1 | 24 | – | – | – | – |
| | 48 | – | – | – | – |
| | 72 | – | – | – | – |
| 2 | 24 | – | – | – | – |
| | 48 | – | – | – | – |
| | 72 | – | – | – | – |
| 3 | 24 | – | – | – | – |
| | 48 | – | – | – | – |
| | 72 | – | – | – | – |
| 4 | 24 | – | – | – | – |
| | 48 | – | – | – | – |
| | 72 | – | – | – | – |
| 5 | 24 | – | – | – | – |
| | 48 | – | – | – | – |
| | 72 | – | – | – | – |
| 6 | 24 | – | – | – | – |
| | 48 | – | – | – | – |
| | 72 | – | – | – | – |

LEGEND: I CORNEAL ULCERATION
II CORNEAL OPACITY
III IRIS INFLAMMATION
IV CONJUNCTIVAL CHEMOSIS (obvious swelling with partial eversion of the lids) and/or pronounced vessel injectio

\+ = POSITIVE RESPONSE
– = NEGATIVE RESPONSE

Prophetic Patch Test

Material is applied to subject's back under the dressing and kept in place for 48 hours. The test site is graded at I5 minutes and 24 hours after the removal of the dressing. Two weeks after the first application a path is left in place for 48 hours, removed and graded I5 minutes and 24 hours later according to the following scale:

SCORING SCALE

| | |
|---|---|
| X | Not patched |
| O | No reaction |
| 0.5 | Minimal reaction |
| 1 | Definite erythema |
| 2 | Erythema with edema |
| 3 | Erythema with vesiculation and edema |
| 4 | Intense Erythema with bullae |
| G | Glazing |
| S | Scaling |

Subjects

100 healthy human adults free of any significant systemic or dermatologic disorder. The area of the back utilized is free of any blemishes which might interfere with grading the test sites. Written informed consent is obtained from are subjects prior to the start of a test.

Prophetic patch test was done on 2 groups of 50 by Derma-test Laboratories Inc., 29-28 4Ist Avenue, Long Island City, N.Y. III01 on the lotion (see Example I, supra) and the cream (see Example II, supra). 0.15 ml of the extracts are used. Also tested was 0.I5 ml petrolatum on I02 subjects using Parke-Davis patch material. In the induction phase, patches were removed and sites scored 48 and 72 hours after application; all test materials were negative at each reading. Challenge patches were applied I2 days after removal of the induction patch readings were made at 48 and 72 hours. After application all patch sites were negative at each reading without any evidence of dermal contact irritation or sensitization.

Virology Studies

Investigation as to the presence of any viruses have been performed on porcine omentum CMFr in a comprehensive viral profile performed by Damon Clinical Laboratory as also described in copending U.S. Patent Application Serial No. 811,505, filed on December 20, I985 by Catsimpoolas and herein incorporated.

## Claims

I. Composition for topical application to balding human skin for reducing terminal hair loss or promoting new hair growth comprising: isolated omentum lipids.

2. Composition according to claim 1 further comprising: a sodium hyaluronate, a silicon, an emulsifier, an antioxidant, a preservative, a hygroscopic agent, and a metal chelating agent.

3. Composition according to claim 2 further comprising: a skin filler and triethanolamine.

4. Composition according to claim 1 wherein said antioxidant comprises: tocopheryl acetate.

5. Composition according to claim I wherein said composition promotes new hair growth on human skin genetically programmed for new hair growth.

6. Composition of claim 5 wherein new hair growth comprises terminal hair.

7. Composition of claim I wherein the omentum lipids are derived from the group consisting of porcine, bovine, ovine, feline omentum lipids and mixtures thereof.

8. Composition of claim I wherein the omentum lipids are isolated using a cryogenic technique.

9. Composition of claim 8 wherein the omentum lipids are isolated by extraction with at least one organic solvent.

10. Composition of claim 9 wherein the extraction is a hexane extraction.

11. Composition of claim 9 wherein the omentum lipids are isolated with a chloroform-methanol extraction.

12. Composition of claim 8 wherein the omentum lipids are isolated by supercritical gas extraction.

13. Composition of claim 12 wherein the gas is $CO_2$.

14. Composition of claim I wherein the omentum lipids are isolated using heat extraction.

15. Composition of claim I wherein the emulsifiers are selected from the group consisting of glycerol stearate, sorbitan stearate, carbomer, polysorbate 60 and mixtures thereof.

I6. Composition of claim I wherein the preservatives are selected from the group consisting of methylparaben, propylparaben, phenoxy-ethanol and mixtures thereof.

17. Composition of Claim 1 wherein the hygroscopic agent is selected from the group consisting of silicone compounds, butylene glycol, hyaluronate compounds, water and mixtures thereof.

18. Composition of claim 17 wherein the silicone compounds are selected from the group consisting of dimethicone, diphenicone, phenyldimethicone, cyclomethicone and mixtures thereof.

19. Composition of claim 1 wherein the chelating agent is EDTA.

20. Composition of claim 1 wherein the skin filler is carbomer 940.

21. Composition of claim I wherein the omentum lipid material is incorporated into a lotion, cream, gel, spray, or stick.

22. Composition of claim I wherein the composition reduces hair loss in humans having common pattern baldness.

23. Composition of claim 21 wherein the lotion, cream ointment, gel, spray or stick consists of a mixture comprising materials selected from the group consisting of omentum lipid material, sodium hyaluronate, sorbitan stearate, propylparaben, polysorbate 60, glyceryl stearate, tocopheryl acetate, dimethicone, carbomer 940, water, butylene glycol, methylparaben, trisodium EDTA, phenoxyethanol, triethanolamine and mixtures thereof.

24. Process for promoting the rate of new hair growth in humans comprising: topically applying a composition containing isolated omentum lipids, sodium hyaluronate, a silicon, an emulsifier, an antioxidant, a preservative, a hygroscopic agent, a metal chelating agent, a skin filler, triethanolamine, water and mixtures thereof to human skin sites having areas genetically programmed for hair follicles that produce terminal hair.

25. Process for minimizing the rate of terminal hair loss in humans, comprising: typically applying a composition isolated omentum lipids, sodium hyaluronate, a silicon, an emulsifier, an antioxidant, a preservative, a hygroscopic agent, a metal chelating agent, a skin filler, triethanolamine, water and mixtures thereof to human skin sites having areas genetically programmed for hair follicles that produce terminal hair.

26. Process of claims 24 or 25 wherein the topical application step further comprises: applying the composition at least once a day to the human skin sites having the areas genetically programmed for hair follicles that produce terminal hair.

27. Process of claim 24 or 25 comprising: using the composition having isolated omentum lipids in a range of from 14 to 18% by weight of the composition.

28. Process of claim 25 further comprising: topically applying a composition having isolated omentum lipids and at least one member of the group consisting of sorbitan stearate, a propylparaben, a polysorbate 60, a glyceryl stearate, a dimethicone, a carbomer 940, water, a butylene glycol, a methylparaben, a trisodium EDTA, a phenoxyethanol, tocopheryl acetate, a triethanolamine, and a mixture thereof to the human skin sites programmed for hair follicles that produce temrinal hair.

Neu eingereicht / Newly
Nouvellement dépos

EXTRACTION OF HxFr FROM CRYOGROUND PORCINE OMENTUM

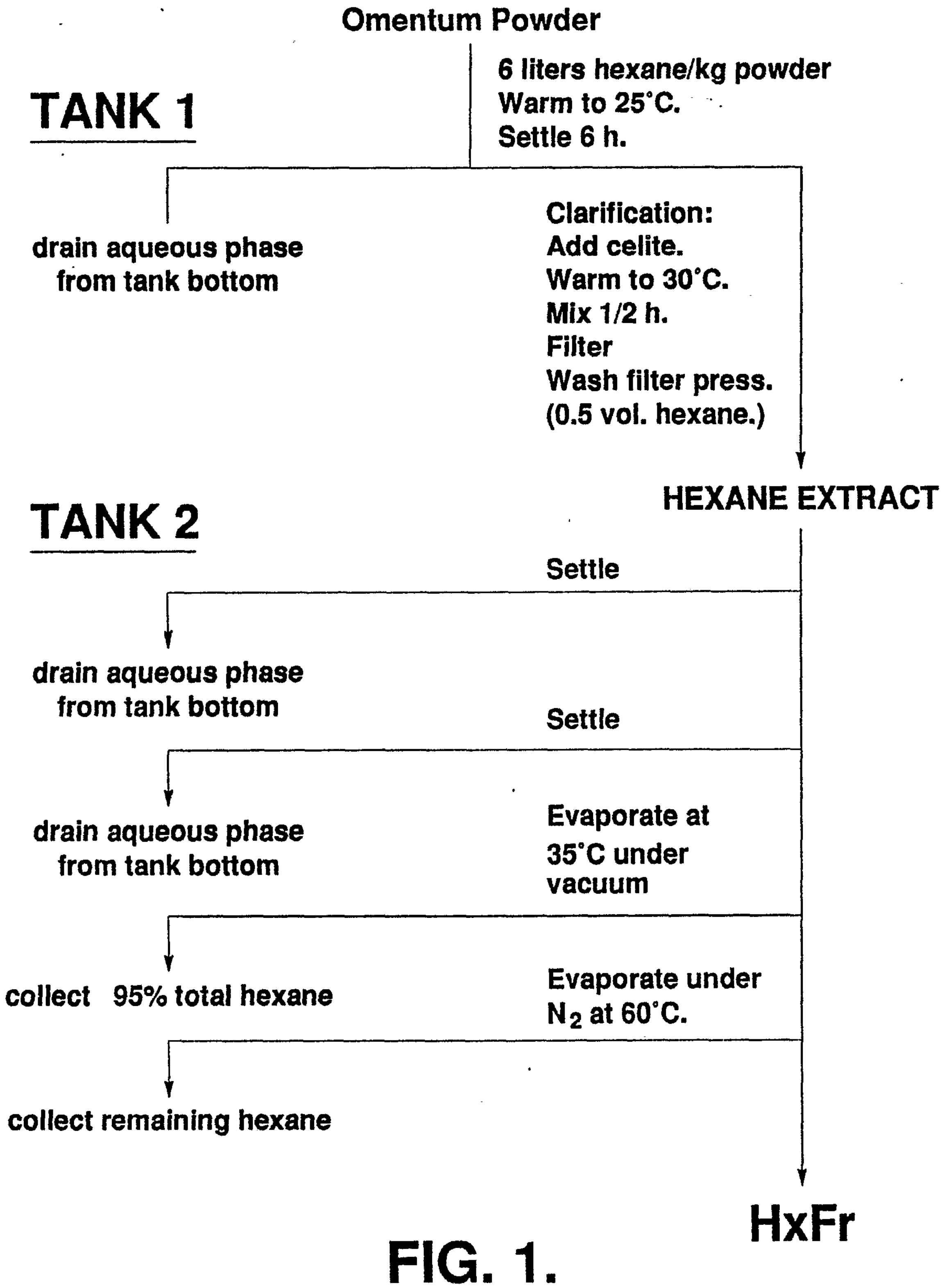


FIG. 1.

Neu eingereicht / N
Nouvellement d

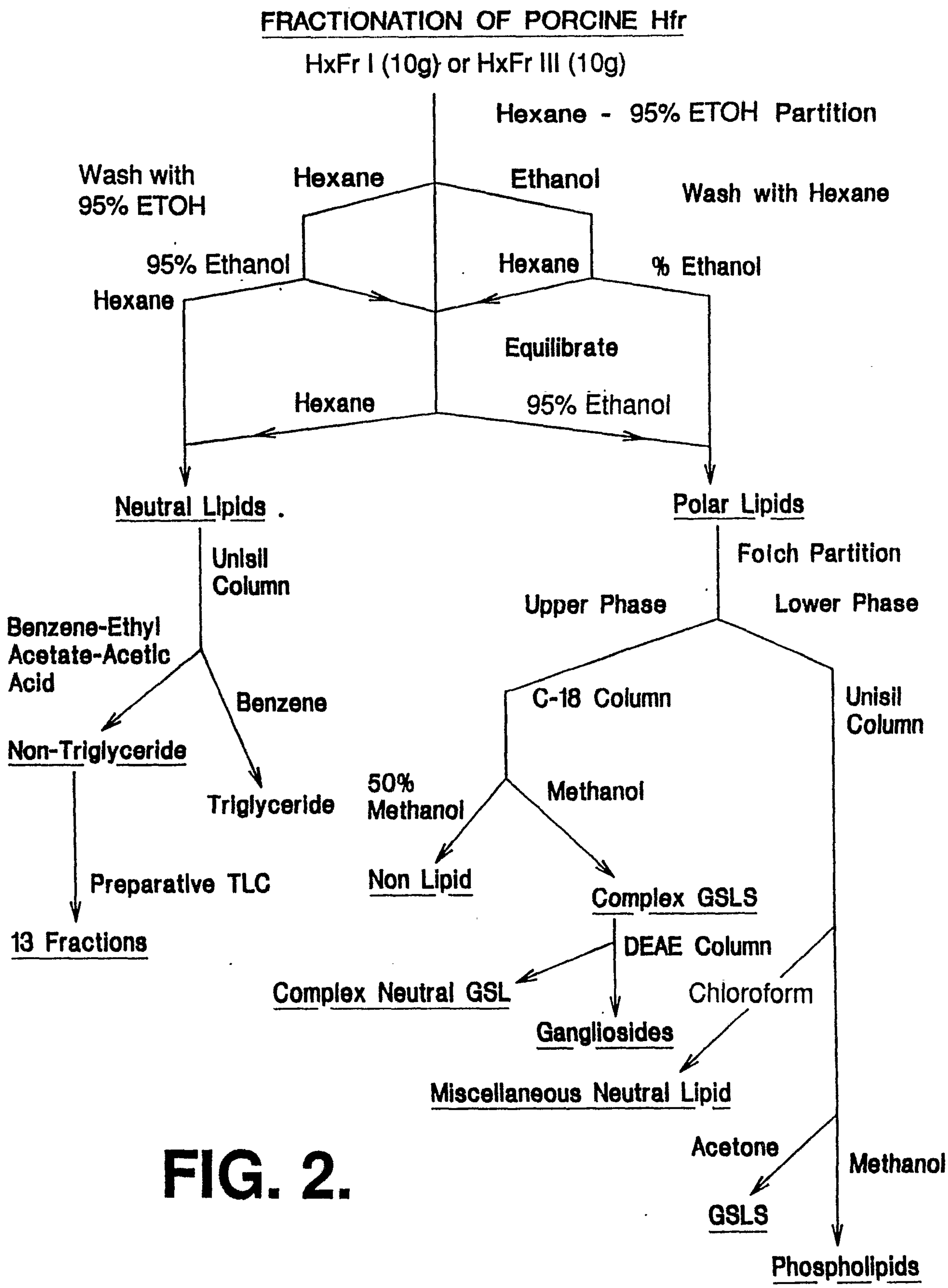


FIG. 2.

icht / Newly filed
ment déposé

Fig.4.

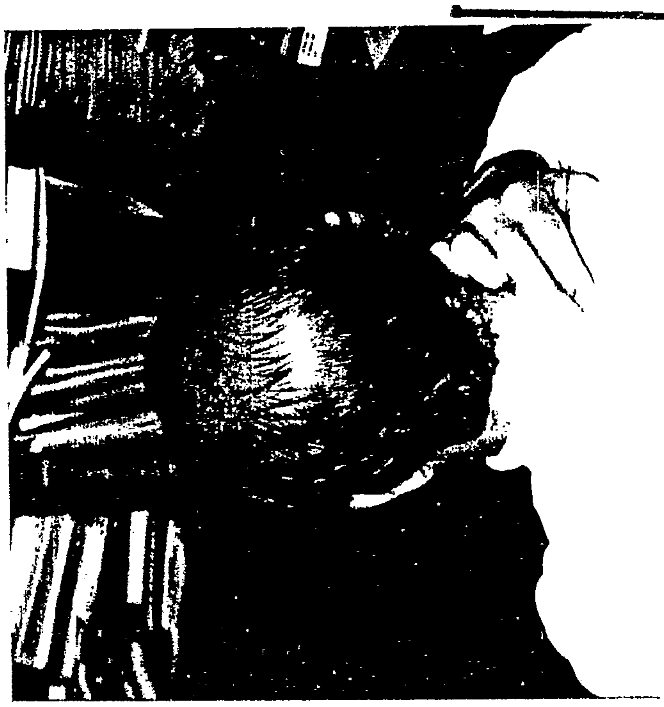

Fig.3.

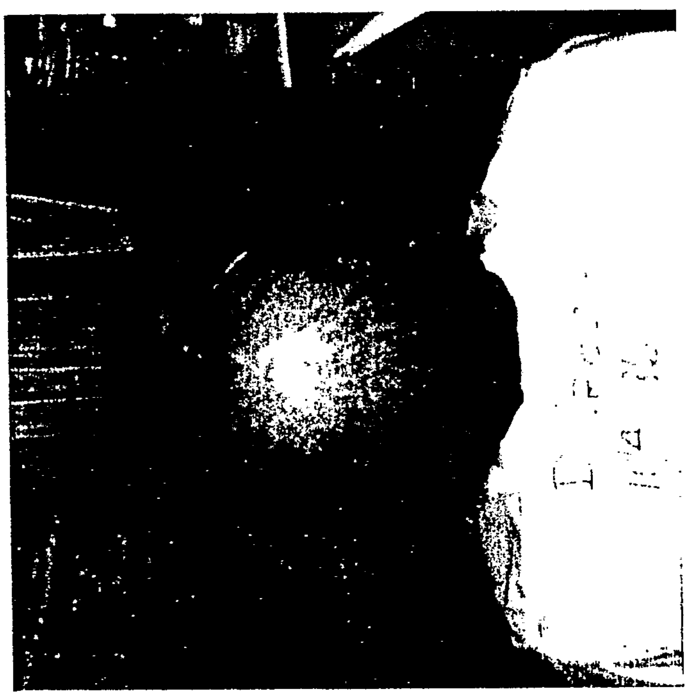

Fig. 6.

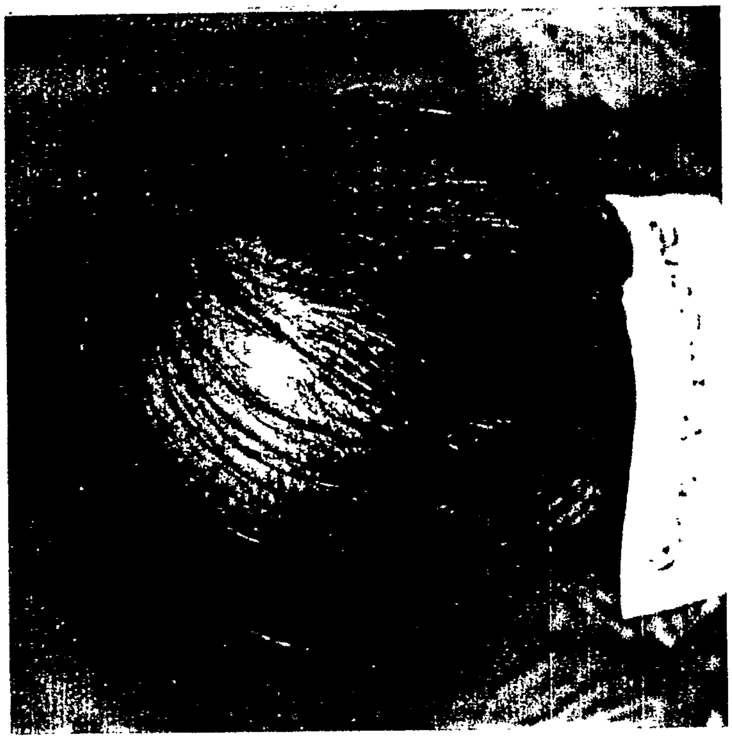

Fig. 5.

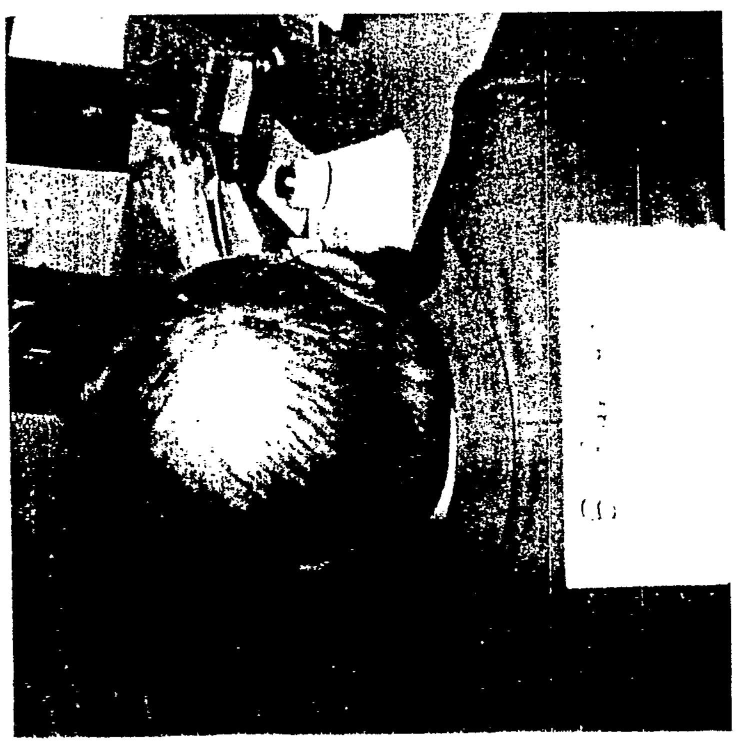

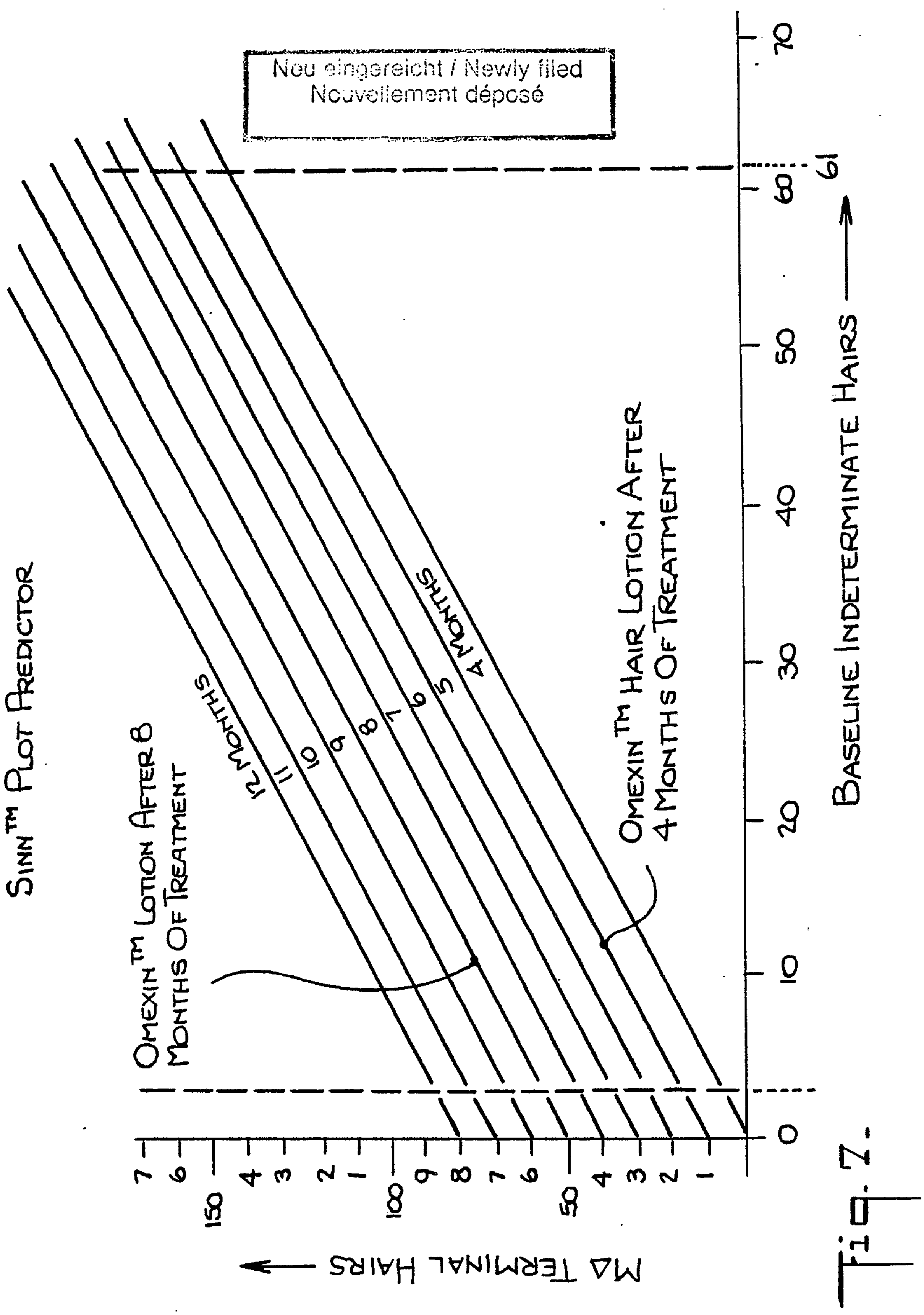
Neu eingereicht / Newly filed
Nouvellement déposé
SINN™ PLOT PREDICTOR
OMEXIN™ LOTION AFTER 8 MONTHS OF TREATMENT
OMEXIN™ HAIR LOTION AFTER 4 MONTHS OF TREATMENT
12 MONTHS
11
10
9
8
7
6
5
4 MONTHS
MΔ TERMINAL HAIRS
BASELINE INDETERMINATE HAIRS
0
10
20
30
40
50
60
61
70
1
2
3
4
50
6
7
8
9
100
1
2
3
4
150
6
7

Fig. 7.

Fig. 8.

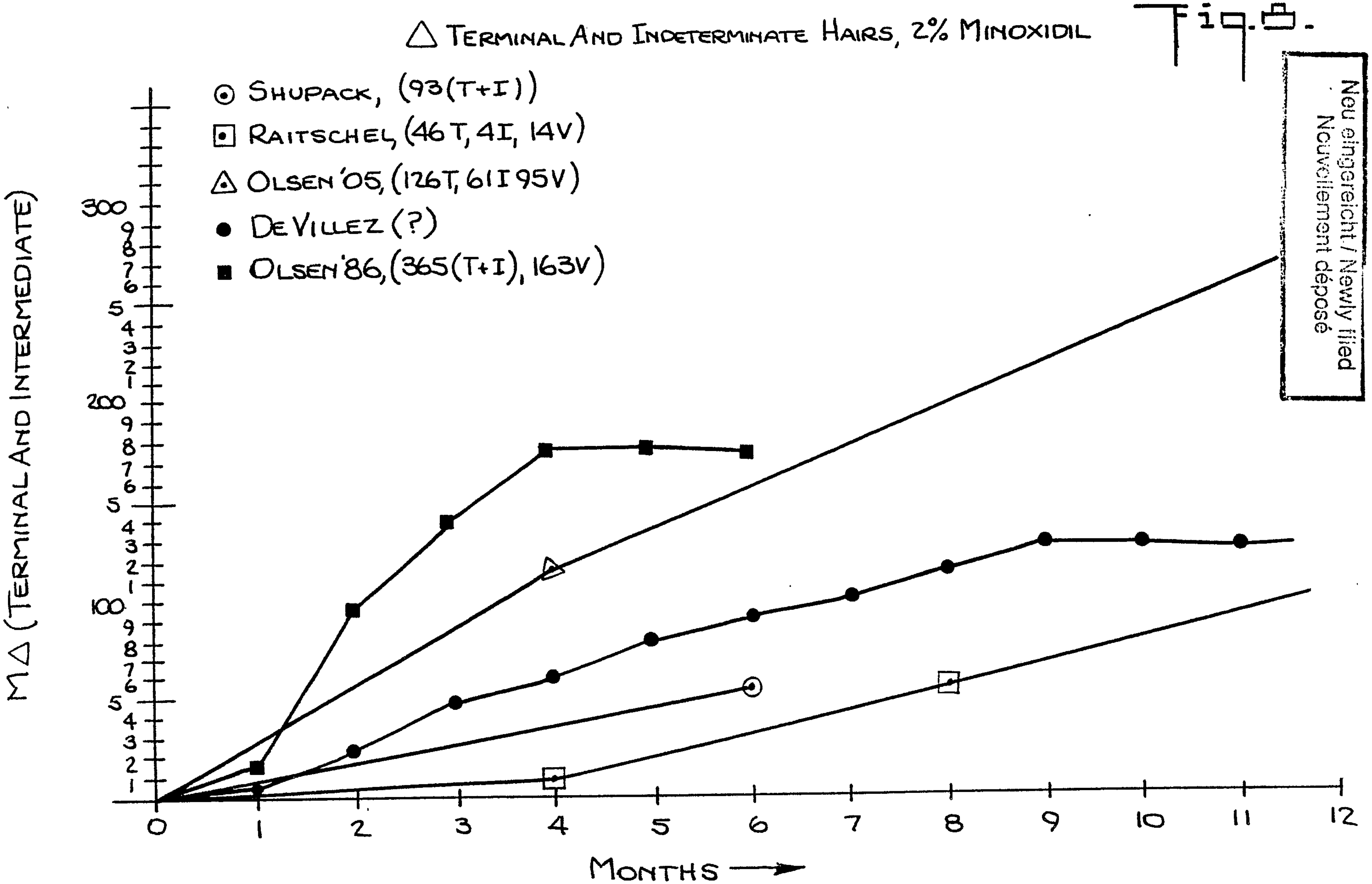


Neu eingereicht / Newly filed
Nouvellement déposé

Neu eingereicht / Newly filed
Nouvellement déposé

Fig. 9.

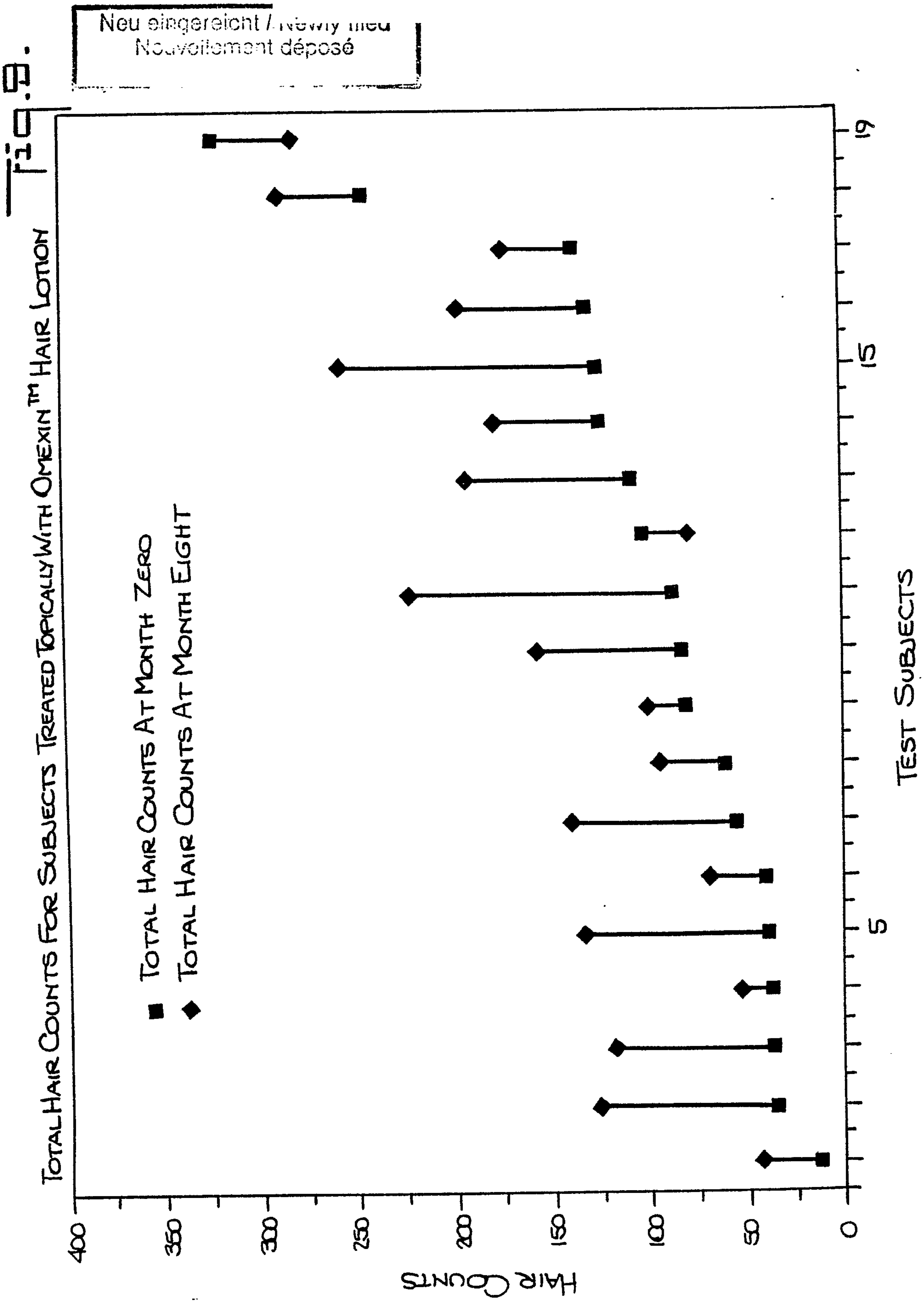

Neu eingereicht / New
Nouvellement dép

Fig.10.